# EUROPEAN PATENT APPLICATION

(11) **EP 2 277 987 A2**
(43) Date of publication of application: **26.01.2011**
(21) Application number: 10011150.9
(22) Date of filing: 17.02.2005
(51) Int. Cl.: C12M 3/00, C12Q 1/68, B01D 15/36, B01J 47/00

(54) **Polyelectrolyte-coated size-exclusion ion-exchange particles**

(30) Priority: 18.02.2004 US 780963; 15.02.2005 US 57936
(62) Divisional of application: 05723310.8
(71) Applicant: Life Technologies Corporation, Carlsbad, CA 92008 (US)
(72) Inventor: Harrold, Michael, P., San Mateo California 94402 (US); Lau, Aldrich, N., K., Palo Alto California 94301 (US); Chen, Shiaw-Min, Fremont California 94539 (US)
(74) Representative: Schnappauf, Georg

(57) **Abstract**

A polyelectrolyte-coated particle, devices for using the particle, methods for using the particle for separating PCR reaction products and/or DNA sequencing reaction products, and compositions for coating the particle are provided.

## Description

### FIELD

The present teachings relate to apparatuses and methods for filtering and/or purifying a sample by using ion-exchange techniques.

### BACKGROUND

Purification of reaction products obtained from, for example, a polymerase chain reaction (PCR) or a sequencing reaction, can present a number of challenges for subsequent, downstream processing. Impurities can cause artifacts in subsequent processing steps. Numerous purification steps to eliminate artifacts can be cumbersome and inefficient. It can be desirable to capture primers, unincorporated nucleotides, primer-dimers, small DNA fragments, and in some cases desalt PCR products. It can be desirable to capture primers, dye-labeled primers, nucleotides, dye-labeled nucleotides, dideoxynucleotides, and dye labeled dideoxynucleotides and desalt DNA sequencing reaction products. A need exists for separation that addresses these and other problems associated with conventional techniques of purification.

### SUMMARY

According to various embodiments, the present teaching provide a particle including a core including ion-exchange material, and a coating including polyelectrolyte material, wherein the core and coating are adapted to separate PCR reaction products. According to various embodiments, the present teaching provide a method for purifying PCR reaction products, the method including providing a plurality of particles, wherein each particle includes a core for ion-exchange and a coating of polyelectrolyte, and contacting the PCR reaction products to separate dsDNA fragments.

According to various embodiments, the present teaching provide a particle including a core including ion-exchange material, and a coating including polyelectrolyte material, wherein the core and coating are adapted to separate DNA sequencing reaction products. According to various embodiments, the present teaching provide a method for purifying DNA sequencing reaction products, the method including providing a plurality of particles, wherein each particle includes a core for ion-exchange and a coating of polyelectrolyte, and contacting the DNA sequencing reaction products to separate dye-labeled ssDNA fragments.

According to various embodiments, the present teaching provide a method for forming a particle, the method including selecting core material and polyelectrolyte material adapted to separating at least one of PCR reaction products and DNA sequencing reaction products, providing the core including ion-exchange material, and coating the core with polyelectrolyte material. According to various embodiments, the present teaching provide a composition including polyelectrolyte material wherein the polyelectrolyte material is adapted to coating ion-exchange material and to providing separation of at least one of PCR reaction products or DNA sequencing reaction products. According to various embodiments, the present teaching provide a system for biological separation, the system including polyelectrolyte material wherein the polyelectrolyte material is adapted to coating ion-exchange material and to providing sieving for separation of at least one of PCR reaction products or DNA sequencing reaction products.

Additional features and advantages of various embodiments will be set forth in part in the description that follows, and in part will be apparent from the description, or may be learned by practice of various embodiments. The objectives and other advantages of various embodiments will be realized and attained by means of the elements and combinations particularly pointed out in the description herein and appended claims.

### BRIEF DESCRIPTION OF THE FIGURES

Fig. 1 illustrates a cross-sectional view of a polyelectrolyte-coated particle, where the coating includes a biopolym

Fig. 2 illustrates a cross-sectional view of a polyelectrolyte-coated particle, where the coating is a synthetic polymer;

Fig. 2a illustrates several synthetic polymers that can be included in the coating for the polyelectrolyte-coated particle.

Figs. 3a-3d demonstrate separation of sequencing reaction products with polyelectrolyte-coated particles with biopolymer in comparison with standard separation techniques, where Figs. 3a-3c demonstrate separation with polyelectrolyte-coated particles, according to various embodiments, Fig. 3d demonstrates separation with an uncoated ion-exchange particle;

Figs. 4a-4b demonstrate separation of PCR reaction products by polyelectrolyte-coated particles with biopolymer, where Fig. 4a illustrates unpurified PCR reaction products including a mixture of a dye-labeled amplicon and a dye-labeled primer, and Fig. 4b illustrates PCR reaction products separated with a polyelectrolyte-coated particle to remove the dye-labeled primer;

Figs. 5a-5b is a set of graphs illustrating a detail of Figs. 4a-4b, respectively;

Fig. 6 demonstrates separation of a sequencing reaction products with polyelectrolyte-coated particles with synthetic polymer;

Figs. 7a-7b demonstrate separation of a sequencing reaction products with polyelectrolyte-coated particles synthetic polymer;

Fig. 8 demonstrates the size cutoff for separation by the polyelectrolyte-coated particles with synthetic polymer for separation using coating polymers with different molecular weights;

Figs. 9a and 9b demonstrate the separation of sequencing reaction products by polyelectrolyte-coated particles with synthetic polymer;

Fig. 10 demonstrates the size-based removal of small dsDNA fragments from larger dsDNA fragments using polyelectrolyte-coated particles with synthetic polymer;

Fig. 11 demonstrates the removal of an oligonucleotide primer from a PCR product using polyelectrolyte-coated particles by illustrating the result of separating components with gel electrophoresis using a 2% agarose gel; and

Fig. 12 demonstrates the DNA size discrimination using non-desalting polyelectrolyte-coated particles.

It is to be understood that the figures are not drawn to scale. Further, the relation between objects in a figure may not be to scale, and may in fact have a reverse relationship as to size. The figures are intended to bring understanding and clarity to the structure of each object shown, and thus, some features may be exaggerated in order to illustrate a specific feature of a structure.

It is to be understood that both the foregoing general description and the following detailed description are exemplary and explanatory only, and are intended to provide an explanation of various embodiments of the present teachings.

### DESCRIPTION OF VARIOUS EMBODIMENTS

The section headings used herein are for organizational purposes only, and are not to be construed as limiting the subject matter described. All documents cited in this application, including, but not limited to patents, patent applications, articles, books, and treatises, are expressly incorporated by reference in their entirety for any purpose.

The term "particle" as used herein refers to an ion-exchange material of liquid, solid, and/or gas that can be coated. The coating can cover the entire exterior surface of the particle or substantial portions thereof. The coating can cover portions of the interior surfaces of the particle. The coating can be irreversible to permanently coat the particle, or reversible to release the particle upon dissolution of the coating. The particle can be a single material or an agglomerate of materials that can be prepared by, for example, fusion, sintering, pressing, compressing, phase separation, precipitation, aggregation and coalescence, or otherwise formed together. The particle can have any shape either regular or irregular such as spherical, elliptical, triangular, cylindrical, etc.

The term "material" as used herein refers to any substance on a molecular level or in bulk and can be a liquid and/or solid, e.g. an emulsion or a resin.

The term "pore size" as used herein refers to a mean measurement, providing a guideline that particles larger than the pore size are less likely to penetrate into the interior of the particle, while smaller particles are more likely to penetrate into the interior of the particle. It is to be understood that the particles admitted to or deflected from a pore are not necessarily exactly the "pore size" given. That is, admittance to or exclusion from the pore is based on many factors, including actual pore size (wherein each pore of a core can have a different size), steric hindrance factors, ionic attractions, polarizations, and the like. Additionally, some particles, such as microporous gel type ion exchange materials, do not have defined pores. The particles have a "pore size that is defined by the intermolecular spacing within the gel matrix to define the size exclusion limit.

The term "ion-exchange" as used herein refers to the process wherein each charge equivalent that can be "coupled" or "captured" on the ion-exchange surface can release an equivalent charge into an appropriate solution. This displacement of counter-ions from the ion-exchange core can release a large number of counter-ions into a sample solution. The selectivity of the ion-exchange core can be greater for the ion to be removed from the sample solution than for the counter-ion of the ion-exchange core. Ions of similar affinity as the counter-ion establish an equilibrium distribution based on the relative affinity of the ions for the ion-exchanger. The equilibrium can either provide or not provide the uptake of ions from solution. The counter-ion can be almost any ion including chloride, hydroxide, acetate, formate, bromide, sulfate, nitrate, phosphate or any other organic or inorganic anion. The choice of counter-ion can be influenced by the nature of the ions in solution that are to be removed. A counter-ion can be selected that has a significantly lower affinity for the ion-exchange core relative to the ion in solution, thus providing exchange with the ion in solution. Neutralization using a cation exchange resin in a mixed bed can drive the uptake of an ion from solution. This can be the case even if the affinity of the cation for the resin is lower than the affinity for the counter-ion. While the above describes the use of anion-exchange particles, the present teachings are analogous for cation-exchange particles. Counter-ions for cation-exchange particles include hydronium, sodium, potassium, ammonium, calcium, magnesium, or any other organic or inorganic cation. Polyelectrolyte-coated ion-exchange particles can be prepared in any ionic form.

The term "mixture" as used herein refers to more than one polyelectrolyte-coated particle used together in a packed column, a mixed-bed, a homogenous bed, a fluidized bed, a static column with continuous flow, or a batch mixture, for example. The mixture can include polyelectrolyte-coated cation-exchange particles, polyelectrolyte-coated anionexchange particles, uncoated cation-exchange particles, uncoated anion-exchange particles, inerts, or any combination thereof. The mixture can include any physical configuration known in the art of separations, and any chemical mixture known in the art of ion exchange. The mixture can be any proportion including stoichiometric equivalent amounts. A mixture of particles can provide size-based removal with desalting of the solution. An example is a polyelectrolyte-coated ion-exchange particle in the hydroxide form in a mixed bed with cation-exchange particles in a hydronium form. A mixture of particles can provide size-based removal of small ions without desalting the solution. An example is a polyelectrolyte-coated ion-exchange particle in the chloride or acetate form (or any other anion other than hydroxide), and no cation exchange material. The choice of counter-ionic form used for the polyelectrolyte-coated ion-exchange particles can be based on the application for which they are to be implemented.

The term "coating" and grammatical variations thereof as used herein refer to less than a monolayer, a monolayer, or multiple layers of a polyelectrolyte with the same charge, or multiple layers of varied polyelectrolytes with opposite charges covering the particle. Smaller molecules, such as, for example, inorganic buffer ions, and nucleotides can penetrate or permeate through the coating and can be retained by or ion-exchanged with the particle. The coating can prevent larger molecules, such as, for example, nucleic acids, from penetrating or permeating through the coating and reacting with the particle.

The terms "polymer," "polymerization," "polymerize," "cross-linked product," "cross-linking," "cross-link," and other like terms as used herein are meant to include both polymerization products and methods, and cross-linked products and methods wherein the resultant product has a three-dimensional structure, as opposed to, for example, a linear polymer. The term "polymer" also refers to oligomers, homopolymers, and copolymers. Polymerization can be initiated thermally, photochemically, ionically, or by any other means known to those skilled in the art of polymer chemistry. According to various embodiments, the polymerization can be condensation (or step) polymerization, ring-opening polymerization, high energy electron-beam initiated polymerization, free-radical polymerization, including atomic-transfer radical addition (ATRA) polymerization, atomic-transfer radical polymerization (ATRP), reversible addition fragmentation chain transfer (RAFT) polymerization, or any other living free-radical polymerization.

The prefix "(meth)acryl" as used herein refers to methacryl and acryl. For example, N-methyl (meth)acrylamide refers to N-methyl methacrylamide and N-methyl acrylamide, and 2-hydroxyethyl (meth)acrylate refers to 2-hydroxyethyl methacrylate and 2-hydroxyethyl acrylate.

The term "DNA" as used herein refers to any nucleic acid, including RNA, PNA, and others as understood to one skilled in the art of molecular biology.

According to various embodiments, polyelectrolyte-coated particles can have many uses such as, for example, in the separation of biomolecules. According to various embodiments, polyelectrolyte-coated particles can provide separation of biomolecules by restricting the ability of large molecules to interact with ion-exchange active sites of the particle. Small molecules that can penetrate into the polyelectrolyte-coated particle can interact with the ion-exchange active sites and can be retained on those sites. Larger, highly charged species can be restricted from interacting with the ion-exchange core by the coating or by the pore size of the core particle. Such larger, highly charged species can remain in solution rather than bind to the ion-exchange particle. Larger species that remain in solution can be separated. Larger molecules are not immobilized on the coating. According to various embodiments, the small molecules can be eluted from polyelectrolyte-coated particles. According to various embodiments, large molecules can include single stranded DNA (ssDNA) fragments, and double stranded DNA (dsDNA) fragments, and small molecules can include nucleotides, short fragments of ssDNA, short fragments of dsDNA, and small ions such as chloride, acetate, and surfactants.

According to various embodiments, a polyelectrolyte-coated particle can be provided by exposing an ion-exchange core to an excess of polyelectrolyte. The core surface can become coated with the polyelectrolyte. The polyelectrolyte can be a biopolymer, including a naturally occurring biopolymer such as DNA, or a synthetic polymer as described herein.

According to various embodiments, a polyelectrolyte-coated particle can be provided by exposing an ion-exchange core to a polyelectrolyte containing charges opposite to that of the core. After the coating of the first polyelectrolyte, the coated particle is exposed to another polyelectrolyte containing charges opposite to that of the first polyelectrolyte. The coating process can be repeated to provide a polyelectrolyte-coated particle with multiple layers of alternative polyanion and polycation.

According to various embodiments, a coating including polyelectrolyte can decrease the interaction of large molecules, including ssDNA, with the core by a size sieving effect. The coating can cover the outer surface of an ion-exchange core, decreasing interaction of large molecules with the surface. The coating can create a size-exclusion barrier decreasing penetration of large molecules into the interior of the core particle. The chemical properties of the polyelectrolyte can determine the sieving properties that the polyelectrolyte-coated particle displays. Properties of the polyelectrolyte such as charge, charge density, hydrophobicity, tactility, flexibility, ratio of monomer units used in co- and ter-polymers, and molecular weight can all be modified in order to provide the desired sieving characteristics. According to various embodiments, the polyelectrolyte coating can be crosslinked in a later step to obtain desirable physical properties and size-exclusion characteristics.

According to various embodiments, a polyelectrolyte-coated particle can function as a size-excluded ion-exchanger by exploiting the inherent porosity of the ion-exchange core. Ion- exchange cores can be obtained with a wide variety of pore sizes, such as 5 angstroms (microporous) and 1000 angstroms or greater (macroporous). An ion-exchange core can be selected based on pore size such that it excludes molecules of a given size based on the requirements of the application. According to various embodiments, the polyelectrolyte coating can be large enough to be excluded from the pores of the ion-exchange core, thereby coating the exterior surface with substantially decreased coating of the interior of the pores. The polyelectrolyte coating can decrease the interaction of large molecules, such as ssDNA with the surface of the ion-exchange core by blocking a substantial amount of the surface ion-exchange sites. The pore size of the ion-exchange core bead can be small enough to decrease the penetration of large molecules, such as ssDNA, into the pores of the core and interacting with the core ion-exchange sites. According to various embodiments, the surface ion-exchange sites can be substantially blocked and the inner ion-exchange sites can become less accessible, such that the polyelectrolyte-coated particle retains significantly less large molecules, such as dsDNA. In contrast, smaller ions such as chloride, acetate, phosphate, pyrophosphate, small oligonucleotides, and nucleotides can enter the pores of the ion-exchange core and interact with interior ion-exchange sites. The coating can decrease the interaction of small ions, like the large molecules, with the surface ion-exchange sites because the surface sites are occupied by the polyelectrolyte coating. The resultant ion-exchange capacity of such a polyelectrolyte-coated particle (for small ions) is equal to the working capacity of the bare ion-exchange core minus the capacity of the surface of the core. The interior pores of the particle provide the substantial ion-exchange capacity of the polyelectrolyte-coated particle after the surface ion-exchange sites have been occupied by the polyelectrolyte coating.

According to various embodiments, a coating can be formed on an ion-exchange core such that the coating has a thickness of from less than an equivalent monolayer to multiple layers. The thickness of the coating can vary over the surface of the ion-exchange core, or the thickness of the coating can be uniform over the entire surface of the ion-exchange core. According to various embodiments, the coating can at least partially cover the ion-exchange core. The coating material can at least partially fill one or more pore or surface feature, for example, pores, cracks, crevices, pits, channels, holes, recesses, or grooves, of the ion-exchange core. For example, an ion-exchange core can be coated on all internal and external surfaces with a polyelectrolyte suitable for forming a coating.

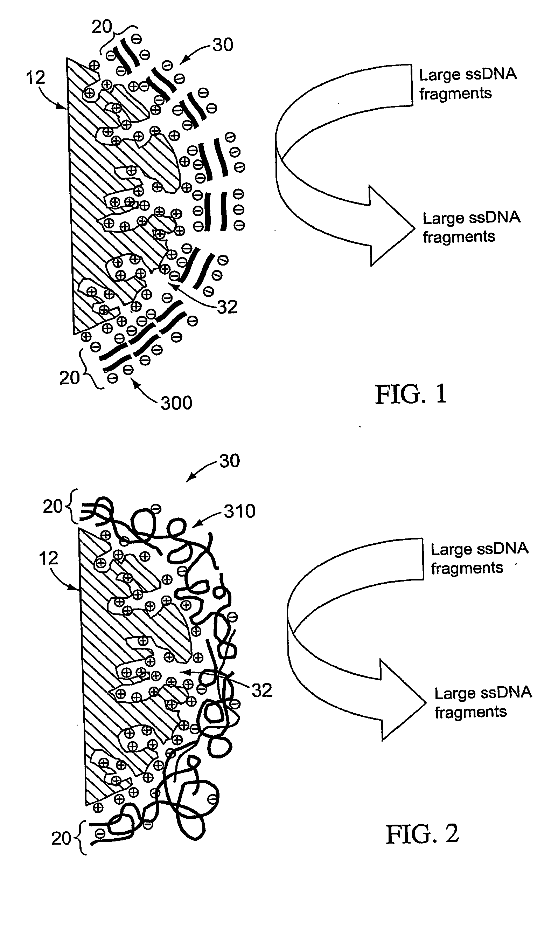

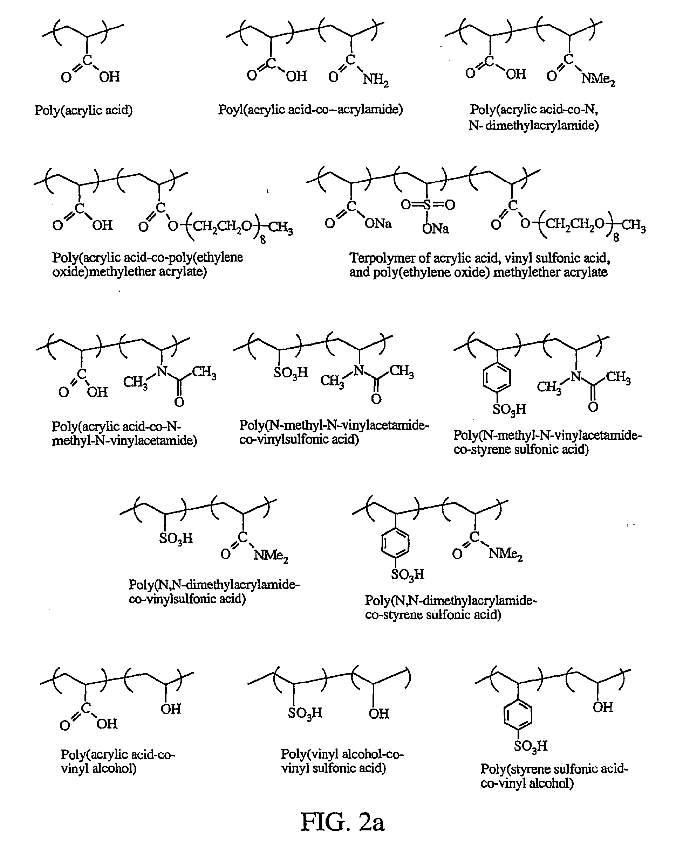

According to various embodiments, Fig. 1 illustrates polyelectrolyte-coated particle 30 which can include ion-exchange core 12 with pores 32 coated with a polyelectrolyte layer 20 composed of a biopolymer 300. Fig. 2 illustrates polyelectrolyte-coated particle 30 which can include ion-exchange core 12 with pores 32 coated with a polyelectrolyte layer 20 composed of a synthetic polymer 310. Small ionic particles (not shown) can sieve and/or enter pores 32 to bind to ion-exchange sites illustrated by positive charges, as in the case of anion-exchange core. The polyelectrolyte coating can substantially decrease the amount of large molecules illustrated by large ssDNA fragments that bind to the ion-exchange core.

According to various embodiments, the ion-exchange core can be an anionic or cationic material. The ion-exchange core can be a polymer, cross-linked polymer, or inorganic material, for example, silica. The ion-exchange core can be a solid core material capable of ion-exchange, or a solid core material treated with an ion-exchange resin. The ion-exchange core can be surface-activated. The ion-exchange core can be non-magnetic, paramagnetic, or magnetic. Exemplary ion-exchange core materials include those listed below.

According to various embodiments, ion-exchange material for the core can include anion-exchange resins such as Macro-Prep High Q, Macro-Prep 25Q, Aminex A-27, AG 1-X2, AG 1-X4, AG 1-X8, and AG 2-X8 (Bio-Rad, Hercules, CA, USA), Chromalite 30 SBG (Purolite Company, Bala Cynwyd, PA, USA), POROS HQ 20 (Applied Biosystems, Framingham, MA, USA), CA08Y and CA08S (Mitsubishi Chemical America, White Plains, NY, USA), Powdex PAO (Graver Technologies, Glasgow, DE, USA), Nucleosil SB (Alltech Associates, Inc., Deerfield, IL, USA), Fractogel TMAE (EM Science, Gibbstown, NJ, USA), IE 1-X8 (Spectrum Chromatography, Houston, TX, USA), Super Q-650S (TosoHaas Bioscience, Montgomeryville, PA, USA), TMAHP-100 (Iontosorb AV, Czech Republic), Chromalite 30 SBA (Purolite International Ltd., UK), and ANEX-QS (Transgenomic, Inc., San Jose, CA, USA). According to various embodiments, the cores material can include PMMA, PS-DVB, silica, and/or cellulose. According to various embodiments, ion-exchange cores can include cation-exchange resins provided by manufacturers similar to those for anion-exchange resins including Chromalite 30 SAG (Purolite International Ltd., UK), AG 50WX8 and Macro-Prep High S (Bio-Rad, Hercules, CA, USA). Other cation and anion resins that can be used as ion-exchange cores will be apparent to one of ordinary skill in the art of ion-exchange resins.

According to various embodiments wherein the ion-exchange core includes a solid core material capable of ion-exchange, the solid core material can be macroporous silica, controlled pore glass (CPG), a macroporous polymer microsphere with internal pores, other porous materials as known to those of ordinary skill in the art of ion-exchange separation, or a combination thereof. The solid core material can have various surface features, including, for example, pores, cracks, crevices, pits, channels, holes, recesses, or grooves. The solid core material can include sodium oxide, silicon dioxide, sodium borate, or a combination thereof. The solid core material can be surface-activated to be capable of ion-exchange, for example, modification to be capable of cation-exchange or anion-exchange. Modification of the solid core material can include treatment of the solid core material to form cationic or anionic substituent groups on the surfaces of the solid core material. As used herein, the term "surface" can include external surfaces and/or internal surfaces. Internal surfaces can be, for example, the surfaces of voids or pores within the solid core material. The solid core material can be surface-activated to include one or more of quaternized functional groups, carboxylic acid groups, sulfonic acid groups, other cationic or anionic functional groups known to those of ordinary skill in the art of ion-exchange separation, or a combination thereof, on the surface of the solid core material.

According to various embodiments, the biopolymer polyelectrolyte can be a naturally-occurring biopolymer such as DNA. Examples of naturally-occurring DNA include sheared salmon sperm DNA, plasmid DNA, restriction digests of plasmid DNA, herring sperm DNA, calf thymus DNA, and other naturally derived DNA. An example of commercially purchased DNA is sheared salmon sperm DNA (Eppendorf AG, Hamburg, Germany). According to various embodiments, the naturally-occurring biopolymer DNA that can be used as a polyelectrolyte in the coating is distinguished as non-sample DNA to indicate that its source is not the sample that has been subjected to the biological reaction.

According to various embodiments, an ion-exchange core can be coated with a synthetic-polymer polyelectrolyte. According to various embodiments, the ion-exchange core can be coated with a water-soluble, or at least slightly water-soluble, polyanion. According to various embodiments, polyanion containing anionic functional groups can be used for coating. The anionic functional group can include carboxylic, boric, sulfonic, sulfinic, phosphoric, or phosphorus group, or a combination thereof. Polyanions containing inorganic acid functional groups can also be used. According to various embodiments, the water-soluble, or at least slightly water-soluble, polyanion can be prepared by copolymerization of an acid- or phenolic-containing monomer, for example, acrylic acid, methacrylic acid, 4-acetoxystyrene that can be hydrolyzed to give phenolic group, 4-styrenesulfonic acid, styrylacetic acid, or maleic anhydride, with a water soluble, at least slightly water-soluble or water-insoluble, co-monomer. According to various embodiments, the synthetic polymer can be can be a homopolymer, a copolymer, a terpolymer, or another polymer.

According to various embodiments, the synthetic polymer can include monomers including: (meth)acrylamide, N-methyl (methyl)acrylamide, N,N-dimethyl (methyl)acrylamide, N-ethyl (meth)acrylamide, N-*n*-propyl (meth)acrylamide, N-*iso*-propyl (meth)acrylamide, N-ethyl-N-methyl (meth)acrylamide, N,N-diethyl (meth)acrylamide, N-hydroxymethyl (meth)acrylamide, N-(3-hydroxypropyl) (methy)acrylamide, N-vinylformamide, N-vinylacetamide, N-methyl-N-vinylacetamide, vinyl acetate that can be hydrolyzed to give vinylalcohol after polymerization, 2-hydroxyethyl (meth)acrylate, 3-hydroxypropyl (meth)acrylate, N-vinypyrrolidone, poly(ethylene oxide) (methy)acrylate, N-(meth)acryloxysuccinimide, N-(meth)acryloylmorpholine, N-2,2,2-trifluoroethyl (meth)acrylamide, N-acetyl (meth)acrylamide, N-amido(meth)acrylamide, N-acetamido (meth)acrylamide, N-tris(hydroxymethyl)methyl (meth)acrylamide, N-(methyl)acryloyltris(hydroxymethyl)methylamide, (methyl) acryloylurea, vinyloxazolidone, vinylmethyloxazolidone, or a combination thereof. According to various embodiments, the synthetic-polymer polyelectrolyte can be poly(acrylic acid-co-N,N-dimethylacrylamide) or poly(N,N-dimethyl acrylamide-co-styrene sulfonic acid).

According to various embodiments, the ion-exchange can be coated with a water-soluble, or at least slightly water-soluble, polycation. According to various embodiments, polycation containing cationic functional groups can be used for coating. The cationic functional group can include protonated primary, secondary, and tertiary amine. According to various embodiments, the water-soluble, or at least slightly water-soluble, polycation can be prepared by copolymerization of a positively charged monomer with a water-soluble, at least slightly water-soluble, or water-insoluble comomer. Examples of polycation can include allyl amine hydrochloride, (3-acrylamidopropyl)trismethylammonium chloride, N-(3-aminopropyl)methacrylamide hydrochloride, and N-vinyl amides that can be hydrolyzed to give an amino group. According to various embodiments, the synthetic polymer can be poly(N-(3-aminopropyl)methacrylamide-co-N,N-dimethylacrylamide).

According to various embodiments, Fig. 2a illustrates the monomeric subunits for those synthetic polymers listed. The abbreviations include acrylic acid (AA), acrylamide (AAm), N.N-dimethylacrylamide (DMA), (polyethylene oxide)monoacrylate (PEOacrylate), and vinyl sulfonic acid (VSA). According to various embodiments, the preparation of synthetic polymer can provide weight average molecular weights (M_{w}) ranging from 50 kiloDaltons to 15.0 megaDaltons, or 200 kiloDaltons to 4.0 megaDaltons, or 500 kiloDaltons to 3.0 megaDaltons. According to various embodiments, the molar percentage of the negatively or positively charged comonomer can contribute from 0.01 percent to 100 percent, or 0.1 percent to 20.0 percent, or 1.0 percent to 10.0 percent.

According to various embodiments, other synthetic polymers can include homopolymers of styrene sulfonic acid, homopolymers and copolymers of acrylic acid, methacrylic acid, vinyl sulfonic acid, styrene sulfonic acid, 4-acetoxystyrene (precursor of 4-hydroxystyrene), and vinylphosphonic acid. According to various embodiments, other synthetic polymers can include homopolymers and copolymers of allyl amine hydrochloride, (3-acrylamidopropyl)trimethylammonium chloride, N-(3-aminopropyl)methacrylamide hydrochloride. The comonomers can include acrylamide, methacrylamide, vinyl acetate that can be converted into vinyl alcohol in a subsequent step, *N,N*-dimethylacrylamide, *N-*ethylacrylamide, *N*-propylacrylamide, *N*-vinyl-*N*-methyl acetamide, 2-hydroxyethyl acrylate, and vinyl methyl ether.

According to various embodiments, the polyelectrolyte can include polyanions such as poly(styrenephosphoric acid), poly(phosphoric acid), homo-polymers and copolymers of maleic acid, derivatives thereof and the like, homo-polymers and co-polymers of fumaric acid, derivatives thereof and the like, peptide-type synthetic polyanions such as poly(aspartic acid), poly(galactronic acid), poly(glutamic acid), nucleic acid type synthetic polyanions such as poly(adenylic acid), poly(inosinic acid), poly(uridylic acid), and natural polyanions such as polysaccharides.

According to various embodiments, the ion-exchange core can be coated with multiple layers of polyelectrolyte. The polyelectrolyte layers can include alternating layers of polyanions and polycations. Such alternating layers can provide strength durability and means to tailor the permeability of the coating. Provided the outer most polyelectrolyte of the multiple-layer structure is negatively charged, DNA fragments will not be immobilized from its solution. Small anions such as, for example, chloride and primers can penetrate or permeate through the multiple-layer structure to be coupled to the core.

According to various embodiments, the polyelectrolyte-coated particles can be provided as a mixture. The mixture can be incorporated in bed or column. According to various embodiments, the polyelectrolyte-coated particles can be provided in a bulk mode. A well-formed chromatographic bed of polyelectrolyte-coated particles is not necessarily required.

According to various embodiments, the polyelectrolyte-coated particles can be provided in a device. The device can be a microfluidic device having one or more pathway, wherein at least a portion of at least one pathway includes polyelectrolyte-coated particles. The device can have an inlet and an outlet in fluid communication with the polyelectrolyte-coated particles. The particles can be present in, for example, a column. As used herein, a column can be in a horizontal or vertical orientation, or in any position between a horizontal and a vertical orientation. The column can include a receptacle such as a cavity, chamber, reservoir, well, reaction region, bed, recess, or other receptacle suitable for containing or retaining polyelectrolyte-coated particles and the reaction products. The column can contain a plurality of polyelectrolyte-coated particles. The outlet of the device can be in fluid communication with a receptacle, such as a purified sample well, a tube, a glass plate, or another means of collecting a purified sample. According to various embodiments, the plurality of polyelectrolyte-coated particles can be a mixture.

According to various embodiments, a device for separating the reaction products can be provided. The device can include a mixture of polyelectrolyte-coated particles. The method can include adding the particles to the column of the device. The reaction products can be placed in or introduced to the inlet of the device.. The reaction products can travel from the inlet through the column including polyelectrolyte-coated particles and optional additional material. The sample can be subjected to a combination of size-exclusion separation and ion-exchange resulting in a filtration and/or purification of the reaction products. The filtered and/or purified solution can be eluted or removed from the column through the outlet, and can be directed to a receptacle for analysis and/or further processing. The reaction products can be moved through the column by centripetal force. According to various embodiments, the plurality of polyelectrolyte-coated particles can be mixed with reaction products in bulk. The plurality of polyelectrolyte-coated particles form a first volume and the reaction products form a second volume where the first volume is less than or equal to the second volume.

According to various embodiments, separation of the reaction products using polyelectrolyte-coated particles can be achieved using a volume of polyelectrolyte-coated particles that is sufficient to provide adequate ion-exchange capacity, such as, ion-exchange of at least 80%, at least 90%, or at least 95% of the reaction products. The separation can occur in ten minutes or less, five minutes or less, or two minutes or less. The separation can include contacting the reaction products with the polyelectrolyte-coated particles for a period of time sufficient for the polyelectrolyte-coated particles to ion-exchange with the reaction products, and removing the purified reaction products from the polyelectrolyte-coated particles.

According to various embodiments, separating the purified reaction products from the particles can include removing the purified reaction products from the polyelectrolyte-coated particles, removing the particles from the purified reaction products, and/or sampling the purified reaction products from the mixture of particles and purified reaction products. An example of sampling the purified reaction products from the mixture of particles and purified reaction products can include analyzing the product in a tube by dipping a capillary directly into the tube and injecting into an instrument for further analysis.

According to various embodiments, separations for the polyelectrolyte-coated particles can include, for example, separation of polymerase chain reaction (PCR) products, separation of DNA sequencing reaction mixtures, and purification of RNA. Polyelectrolyte-coated particles can also be used for purification and/or separation of, for example, oligonucleotides, ligase chain reaction products, proteins, antibody binding reaction products, oligonucleotide ligation assay products, hybridization products, and antibodies. Polyelectrolyte-coated particles can also be used for desalting of biological products or reaction mixtures.

According to various embodiments, the selectivity of the polyelectrolyte-coated particles can depend on at least one of these criteria: (1) the molecular weight of the polyelectrolyte in the coating, (2) the chemical nature of the charged functionality and the charge density or molar percent of the charge of the polyelectrolyte in the coating, (3) the pore size of the ion-exchange core, (4) the nature of co-monomer in the coating polymer, and (5) the ratio of various co-monomers in the polymer. Each separation can provide different desirable features for at least one of the above criteria.

According to various embodiments, PCR products include materials that can interfere with downstream analysis. PCR products can include amplified target sequences (amplicons), buffer salts, surfactants, metal ions, enzymes (e.g. polymerase), nucleotides, oligonucleotide primers, and other components in solution. According to various embodiments, the target sequences of double-stranded DNA (dsDNA) can be analyzed, or used in subsequent enzymatic reactions that can be sensitive to at least some of the other PCR products. For example, free nucleotides and oligonucleotide primers can interfere with downstream enzymatic reactions. According to various embodiments, a polyelectrolyte-coated particle can separate nucleotides, oligonucleotide primers, and buffer salts from the target sequences of dsDNA. The resulting solution can contain purified PCR products in a desalted environment, and can be used in downstream reactions and analyses.

According to various embodiments, PCR purification can be directed toward separating larger double stranded DNA (dsDNA) from smaller ssDNA, and dsDNA (e.g. primer-dimer, an unwanted side reaction product which is a dsDNA, or other non-specifically amplified fragments), free nucleotides, and salts. According to various embodiments, PCR products for removal include nucleotides, primers with 45 nucleotides of ssDNA, primer-dimer with 60 bp of dsDNA, and dsDNA fragments smaller than 200 bp. According to various embodiments, primers, primer-dimer, and DNA fragments smaller than 100 bp can be captured by polyelectrolyte-coated particles. According to various embodiments, primers, primer-dimer, and DNA fragments smaller than 300 bp can be captured by polyelectrolyte-coated particles.

According to various embodiments, separation of larger dsDNA from other PCR products can include desalting. According to various embodiments, separation of larger dsDNA from other PCR products does not include desalting. There are circumstances when desalting can interfere with downstream processing such as separation and detection of the larger dsDNA PCR products.

According to various embodiments, the polyelectrolyte-coated particles can be subjected to the same PCR conditions as the PCR reactants prior to PCR termination and purification. The polyelectrolyte-coated particles can be subjected to temperature cycling from 65 to 95 °C. The polyelectrolyte-coated particles can be bundled into a device that provides PCR and subsequent purification.

According to various embodiments, an ion-exchange core with a pore size in the range of 100 Angstroms to 2000 Angstroms, coated with a polyelectrolyte of M_{w} in the range of 1.0 megaDaltons to 3.0 megaDaltons provides PCR purification. According to various embodiments, an ion-exchange core with a pore size of 1000 Angstroms, coated with a polyelectrolyte of M_{w} of 1.7 megaDaltons to 2.6 megaDaltons provides PCR purification.

According to various embodiments, DNA sequencing reaction products can include material that can interfere with downstream analysis. The quality of separation for purification sequencing reaction products can be evaluated by analyzing at least one of these criteria: (1) residual dye artifacts ("blobs") that appear as broad peaks superimposed over the sequence data, (2) peak intensity balance between large or small fragments, and (3) desalting of the sequencing reaction products.

According to various embodiments, DNA sequencing reaction products can include dye-labeled target sequences (the "sequencing ladder"), buffer salts, phosphate and pyrophosphate ions, metal ions, enzymes (e.g. polymerases), nucleotides, oligonucleotide primers, dye-labeled oligonucleotide primers, and other components such as residual, unincorporated dye-labeled-dideoxynucleotides ("dye terminators"). According to various embodiments, the dye-labeled target sequences can be subjected to electrophoretic analysis and DNA sequencing ("basecalling") that can be sensitive to at least some of the other sequencing reaction products resulting in "blobs" that can cause errors in "basecalling." According to various embodiments, capillary sequencers can use electrokinetic injection to introduce sequencing reaction products into the capillary for electrophoretic separation. The presence of salts in the sequencing reaction products can affect their introduction into the capillary, where a reduced salt concentration can enhance injection into the capillary.

According to various embodiments, polyelectrolyte-coated particles can remove material that produce "blobs" from and desalt sequencing reaction products. According to various embodiments, sequencing reaction products purified with polyelectrolyte-coated particles can have a salt concentration of less than or equal to 100 µM or less than or equal to 50 µM. A sample solution purified by polyelectrolyte-coated particles can be suitable for electrokinetic capillary injection. For these and other purposes, the polyelectrolyte-coated particle can have a size-exclusion limit of, for example, less than 10 nucleotides ssDNA, and can be able to remove small ions such as salts and dye-labeled primers from a sample solution while leaving ssDNA free in solution. Sequencing reaction purification using polyelectrolyte-coated particles can be used to separate ssDNA, for example, having a size of from 10 nucleotides to 1500 nucleotides or larger in size, from smaller components such as, for example, dye-labeled primers and salts.

According to various embodiments, separation of DNA sequencing reaction products can include separating primers, dye-labeled primers, and salts from dye-labeled ssDNA targets by substantially excluding dye-labeled ssDNA fragments having greater than 45 nucleotides.

According to various embodiments, purification of a sequencing reaction sample can remove dye-labeled dideoxynucleotides and salts from the sequencing reaction products by allowing such components to pass through the coating and react with the ion-exchange core, leaving a purified sample containing an amount of ssDNA relative to the pre-filtered amount, in an amount of 70% or more, 80% or more, 90% or more, or 95% or more.

According to various embodiments, an ion-exchange core with a pore size in the range of 5 Angstroms to 1000 Angstroms, coated with a polyelectrolyte of M_{w} in the range of 1000 Daltons to 6.0 megaDaltons provides sequencing reaction purification. According to various embodiments, an ion-exchange core with a pore size of 10 Angstroms to 50 Angstroms, coated with a polyelectrolyte of M_{w} of 2.4 megaDaltons to 4.9 megaDaltons provides sequencing reaction purification.

According to various embodiments, a method for purifying DNA sequencing reaction products can include providing a plurality of polyelectrolyte-coated particles, and contacting the DNA sequencing reaction products to separate dye-labeled ssDNA fragments. The method can include removing residual dye artifacts such as dye-labeled primers that can result in blobs in the sequencing analysis. The method can include maintaining dye-labeled ssDNA fragment lengths.

### EXAMPLES

### COATING PARTICLES WITH BIOPOLYMER:

The ion-exchange resin was converted to an ion-exchange by washing a 100uL volume of resin with 1000uL of 1M salt, acid, or base solution. The mixture was vortexed for 5 minutes, and spun down in a centrifuge. The supernatant was removed and another 1000uL aliquot of salt, acid, or base solution was added. This was repeated three times. The ion-exchange core was then washed and spun five times using 1000uL aliquots of DI water.

The ion-exchange cores were coated with DNA by repeated washings with 100uL aliquots of 1mg/mL sheared salmon sperm DNA (Eppendorf AG, Hamburg, Germany). Coating was performed by washing a 100uL volume of resin with 100uL of 1mg/mL sheared salmon sperm DNA. The mixture was vortexed for 5 minutes, and spun down in a centrifuge. The supernatant was removed and another 100uL aliquot of 1mg/mL sheared salmon sperm DNA was added. This was repeated two times. The SEIE particles were then washed and spun three times using 1000uL aliquots of DI water.

### COATING PARTICLES WITH SYNTHETIC POLYMER:

Poly(AA-co-DMA) polyelectrolyte for coating particles was provided by free radical polymerization of 0.32 g (4.44 mmol) of acrylic acid with 8.04 g (81.07 mmol) of N,N-dimethylacrylamide in 200 mL of DI water at 45 °C for 15 hours, using ammonium persulfate as an initiator and N,N,N'N'-tetramethylethylenediamine as a catalyst. The resulting polymer was purified by dialysis (50K MWCO) and lyophilization to provide 7.70 g (92 % yield) of the polymer; M_{w} = 3.40 MDa, Mn = 2.67 MDa.

Prior to coating, the anion-exchange resin was first converted into hydroxide anion-exchange core in the same manner as the previous example. A 1000 µL aliquot of DI water was added to 0.20-0.25 mL volume of an anion-exchange core in chloride form. The mixture was vortexed for 2 minutes, and spun down in a centrifuge. The supernatant was removed and this DI water washing was repeated two times. A 1000 µL aliquot of 2.0 M of ammonium hydroxide was added to the washed resin. The mixture was vortexed for 2 minutes, let standing for 5 minutes at ambient temperature, vortexed one minute, and spun down in a centrifuge. The supernatant was removed and this ammonium hydroxide washing was repeated two times. A 1000µL aliquot of DI water was added to the pellet of ion exchange particles, vortexed for 1 minute, spun down by a centrifuge, and supernatant removed. This final DI water washing was repeated one more time. The resin was redispersed in 500 µL of DI water in a snap-capped polypropylene micro-centrifuge tube and stored in a refrigerator prior to use.

To a 1.5 mL microcentrifuge tube containing 15-20 µL of the wet anion exchange resin, 1.0 mL of the polymer solution (0.5 weight percent solution in deionized water) was added. It was vortexed for 1 minute, let standing at ambient temperature for 5 minutes, vortexed for additional one minute, spun down in a centrifuge, and supernatant removed. The polymer solution coating was repeated one more time. The pellet was then washed with 1 mL of DI water and spun down three times. The final washed resin was re-dispersed in 500 µL of DI water and stored in a refrigerator prior to use.

Alternatively, poly(AA-co-DMA) polyelectrolyte for coating particles was provided by polymerization under inert atmosphere (ultra pure nitrogen). To a 500-mL round bottom three-neck flask equipped with a 2" Teflon stirring blade, a glass bleeding tube for purging, and a water-cool condenser, was charged with 150.0 mL of Milli-Q water, 8.0160 g (80.86 mmol) of re-distilled *N,N*-dimethylacrylamide (Dajac), 0.3285 g (4.56 mmol) of redistilled acrylic acid (Aldrich Chemical) and 0.8043 g of an 1.9972 wt% aqueous solution of ammonium persulfate. This mixture was purged with ultra pure nitrogen at 150 mL/min for 60 minutes while stirred at a constant speed of 200 rpm. To purged solution, 80 µL of *N,N,N'N'*-tetramethylethylenediamine (Electrophoresis reagent from Aldrich Chemical) was added. The mixture was lowered into an oil bath at 50±1 °C and stirred at 200 rpm for a period of 3.5 hours. At the end of the reaction time, 50 mL of DI water was added and stirred for 5 minutes. The resulting water-clear solution was dialyzed with 50K MWCO Spectra/Pro membrane in 5 Gal of DI water for 4 days, with water changed twice every 24 hours. The dialyzed solution was lyophilized to give 7.70 g (92.0 % yield) of copolymer. Molecular weight was determined by GPC/MALLS to be 2.4 MDa Mw and 1.26 MDa Mn.

### DNA SEQUENCING REACTION PURIFICATION BY POLYELECTROLYTE-COATED PARTICLES WITH BIOPOLYMER:

For DNA sequencing reaction purification, a sample was prepared containing 400 uL dRhodamine Terminator Ready Reaction Mix (Applied Biosystems, Foster City, CA, USA), 50 uL M13 universal reverse primer (3.2 pmol/uL), 25 uL template-amplicon (∼100 ng/uL), and 525 uL DI water. This solution was aliquoted into wells in a thermal cycler plate at a volume of 20uL/well. The mixture was subjected to 25 cycles of heating, wherein each cycle included heating at 95°C for 10 seconds, heating at 50°C for 5 seconds, and heating at 60°C for 120 seconds.

To provide the polyelectrolyte-coated particles with a biopolymer, 100uL each of Aminex A-27, Bio-Rad AG 1-X8, and Bio-Rad AG 2-X8 were prepared as hydroxide polyelectrolyte-coated particles as described above. The resins were coated with 1mg/mL sheared salmon sperm DNA as described above and washed with DI water. 100uL of each of the coated anion exchange particles was mixed with 100uL of Chromalite 30 SAG, prepared as hydrogen-form polyelectrolyte-coated particles, as described above, to form a mixed ion exchange bed. The mixed beds were washed with 1mg/mL sheared salmon sperm DNA as described above and washed with DI water. 2uL of the each of the coated mixed beds were added to a small MicroAmp® tube (Applied Biosystems, Foster City, CA, USA). A mixed bed prepared from uncoated Aminex A-27 and Chromalite 30 SAG was prepared as a control.

To perform the purification, 1uL of the above described sequencing reaction was added to each MicroAmp tube containing 2uL of the DNA-coated mixed bed ion exchange resins. The tubes were vortexed for 5 minutes after which 5uL DI water was added to each tube and mixed with a pipette. The microcentrifuge tubes were spun at 5000x g and 5 µL of supernatant was removed from each tube and pipetted into a 96 well plate for analysis on a ABI Prism® 3100 sequencer. The samples were analyzed on the ABI Prism® 3100 using a 36 cm array, POP6® polymer (Applied Biosystems), and a standard sequencing module.

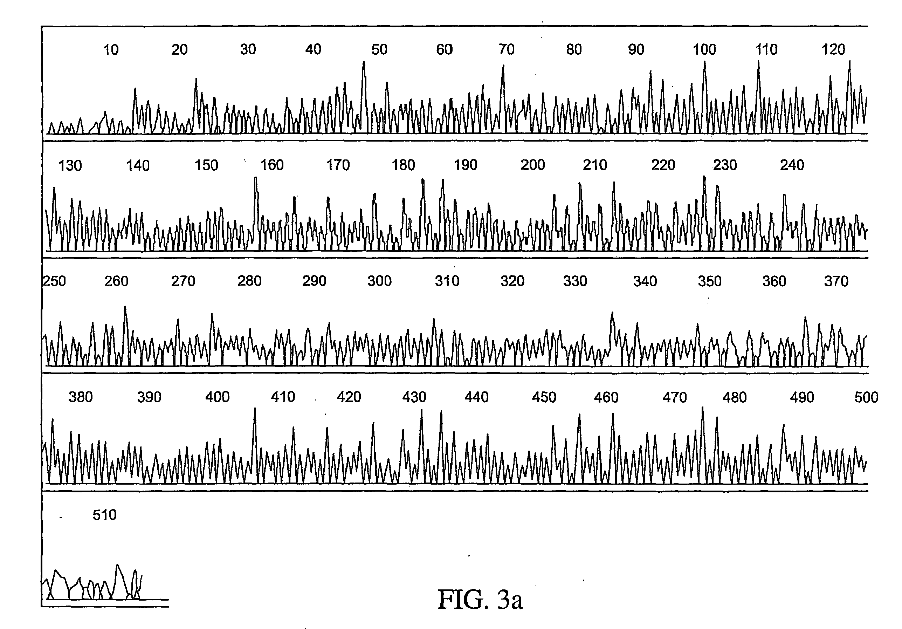

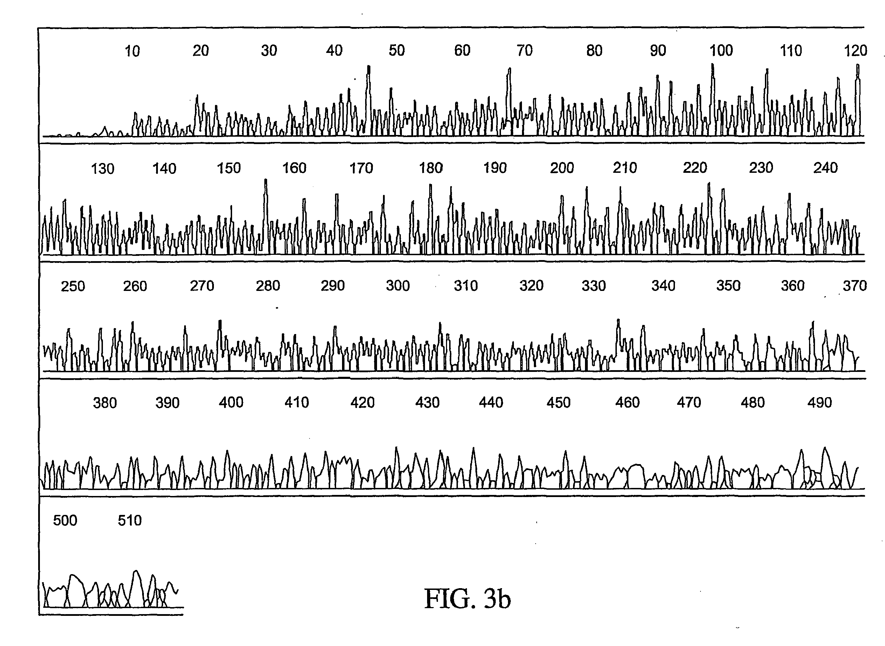

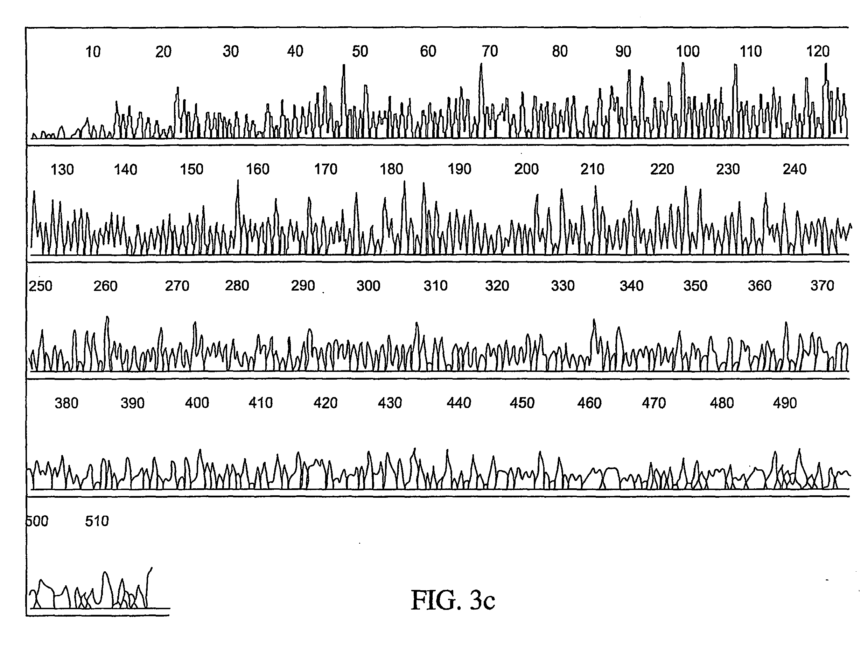

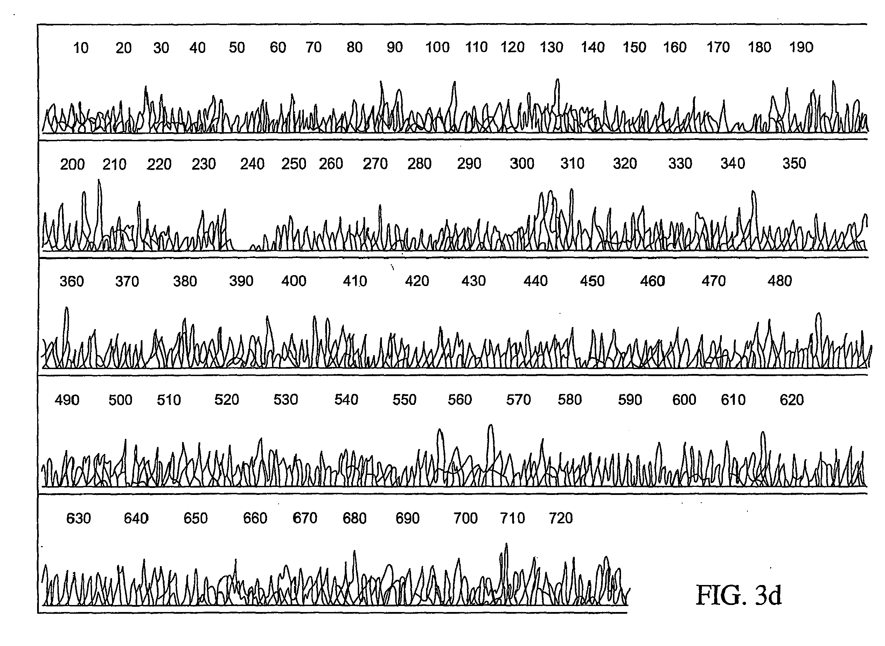

Figs. 3a-3d illustrate the results from this purification. Figs. 3a-3c illustrate that the biopolymer-polyelectrolyte-coated particles provided desirable purification (Fig. 3a illustrating DNA-coated Aminex A-27, Fig. 3b illustrating DNA-coated Bio-Rad AG 1-X8, and Fig. 3c illustrating DNA-coated Bio-Rad AG 2-X8, all three in a cationic-anionic mixed bed). For example, the substantial removal of dye blob (residual dye-labeled ddNTPs) and the relatively high signal strength indicated a desirable desalting of the sample. By contrast, Fig. 3d illustrates that the uncoated mixed bed (Aminex A-27 mixed with Chromalite 30 SAG) did not provide desirable purification. Loss of most of the DNA fragments was observed as is expected from purification with an uncoated anion exchange resin.

### PCR REACTION PURIFICATION BY POLYELECTROLYTE-COATED PARTICLES WITH BIOPOLYMER:

To demonstrate PCR reaction purification, a sample was prepared using a fluorescently-labeled primer so that the PCR product could be analyzed on a fluorescent capillary sequencer. A solution was prepared containing 102 uL PCR master mix (Applied Biosystems), 4 uL FAM-labeled forward primer (20 pmol/uL), 4 uL reverse primer (20 pmol/uL), 20 uL human gDNA, CEPH 1347-02 (50 ug/uL), and 70 uL deionized water (DI). This solution was aliquoted into wells in a thermal cycler plate at a volume of 20uL/well. The mixtures were heated at 96°C for 5 minutes, followed by 40 cycles of heating, wherein each cycle included heating at 96°C for 30 seconds, then at 60°C for 120 seconds.

Particles coated with a biopolymer, similar to the previous example were provided. To perform the purification, 1uL of the above described solution was added to a MicroAmp tube containing 2uL of the DNA-coated mixed bed ion exchange resins. The tube was vortexed for 5 minutes after which 5uL DI water was added to the tube and mixed with a pipette. The microcentrifuge tube was spun at 5000x g and 5 µL of supernatant was removed, added to 5uL deionized formamide, and pipetted into a 96 well plate for analysis on a ABI Prism® 3100 sequencer. The samples were analyzed on the ABI Prism® 3100 using a 36 cm array, POP-6® capillary electrophoresis polymer (Applied Biosystems), and a standard fragment analysis module.

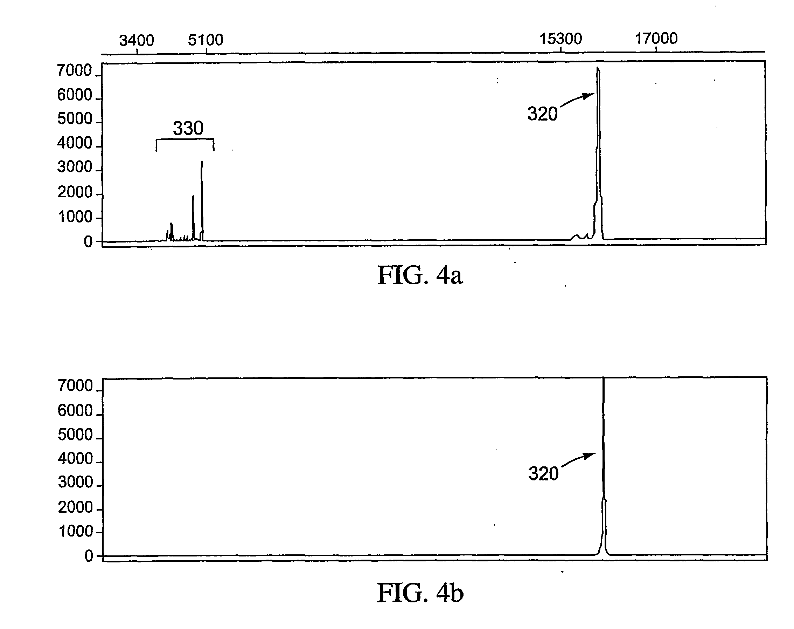

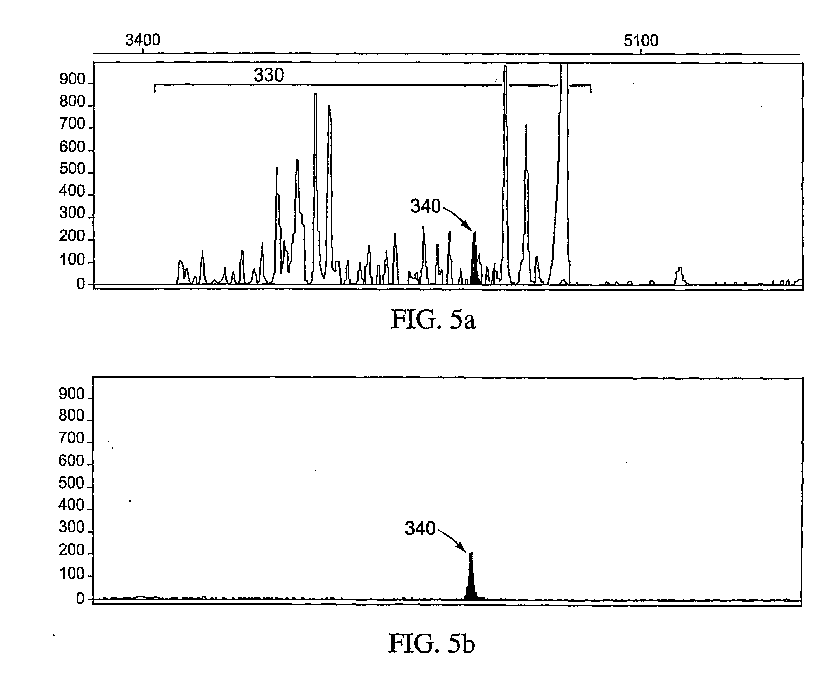

Figs. 4 and 5 illustrate the results from this purification as analyzed using GeneScan® software. The experiment was designed to observe the removal of the majority of a dye-labeled primer from a 550bp PCR product while retaining a double stranded DNA amplicon. Fig. 4a illustrates the PCR reaction solution prior to purification. Fig. 4b illustrates the PCR reaction solution purified and desalted by polyelectrolyte-coated particles. Fig. 4a illustrates peaks from 4000-5000 scans, labeled 330, generated by the primer, while the peak at 15,500 scans, labeled 320, was generated by the 550bp amplicon. The presence of PCR primer can often interfere with subsequent analyses of the PCR product or with subsequent reactions. Removal of the primer from PCR product can be desirable. Fig. 4b illustrates the resulting data when the PCR product is purified as described in the previous paragraph using polyelectrolyte-coated particles with biopolymer. The 550bp amplicon, labeled 320, is still visible, but the primer has been reduced to a less than detectable amount.

Figs. 5a and 5b are a magnified view of a region of the electrophorograms illustrated in Figs. 4a and 4b, focusing on the region of the PCR primer, labeled 330. An internal standard was added to the injection solution after the purification step, but prior to analysis on the ABI Prism® 3100. The internal standard, a synthetic ROX-labeled oligonucleotide at 20nM concentration, was used to measure the relative injection efficiency from the two solutions. The internal standard was observed in both electrophorograms, labeled 340. Fig. 5 illustrates that the injection efficiency from the two solutions is similar. Using the internal standard to normalize the peak heights, the reduction in primer is 100%, while the loss of PCR product is 22%. This example illustrates that contact with the polyelectrolyte-coated particles can substantially decrease the primer from the PCR solution while leaving 78% of the PCR product in solution. Contact of the same solution with uncoated ion exchange beads resulted in loss of most of the primer as well as all of the PCR product (not shown).

### DNA SEQUENCING REACTION PURIFICATION BY POLYELECTROLYTE-COATED PARTICLES WITH SYNTHETIC POLYMER:

For DNA sequencing reaction purification, a sample was prepared using a fluorescently-labeled primer so that the sequencing reaction product could be analyzed on a fluorescent capillary sequencer. A solution was prepared containing 400 uL dRhodamine Terminator Ready Reaction Mix (Applied Biosystems, Foster City, California, 50 uL M13 universal reverse primer (3.2 pmol/uL), 25 uL template-amplicon (∼100 ug/uL), and 525 uL DI water. This master solution was aliquoted into wells in a thermal cycler plate at a volume of 20uL/well. The mixture was subjected to 25 cycles of heating, wherein each cycle included heating at 95°C for 10 seconds, heating at 50°C for 5 seconds, and heating at 60°C for 120 seconds.

To provide the polyelectrolyte-coated particles coated with a synthetic polymer, 20uL of Bio-Rad Macro-Prep High Q ion exchange resin was prepared as hydroxide polyelectrolyte-coated particles as described herein. The resin was coated with poly(acrylic acid-co-N,N-dimethylacrylamide), hereafter referred to as poly(AA-co-DMA), as described herein and washed with DI water. 20uL of poly(AA-co-DMA)-coated Macro-Prep High Q was mixed with 20uL of Chromalite 30 SAG (as hydrogen polyelectrolyte-coated particles, as described herein). 5uL of the ion exchange particle mixture was added to a small MicroAmp® tube (Applied Biosystems, Foster City, CA, USA).

To provide purification, 1uL of the above described sequencing reaction was added to a MicroAmp tube containing 5uL of the DNA-coated mixed bed ion exchange resins. The tube was vortexed for 5 minutes after which 5uL DI water was added to the tube and mixed with a pipette. The microcentrifuge tube was spun at 5000x g and 5 µL of supernatant was removed and pipetted into a 96 well plate for analysis on a ABI Prism® 3100 sequencer. The samples were analyzed on the ABI 3100 using a 36 cm array, POP-6® (Applied Biosystems), and a standard sequencing module.

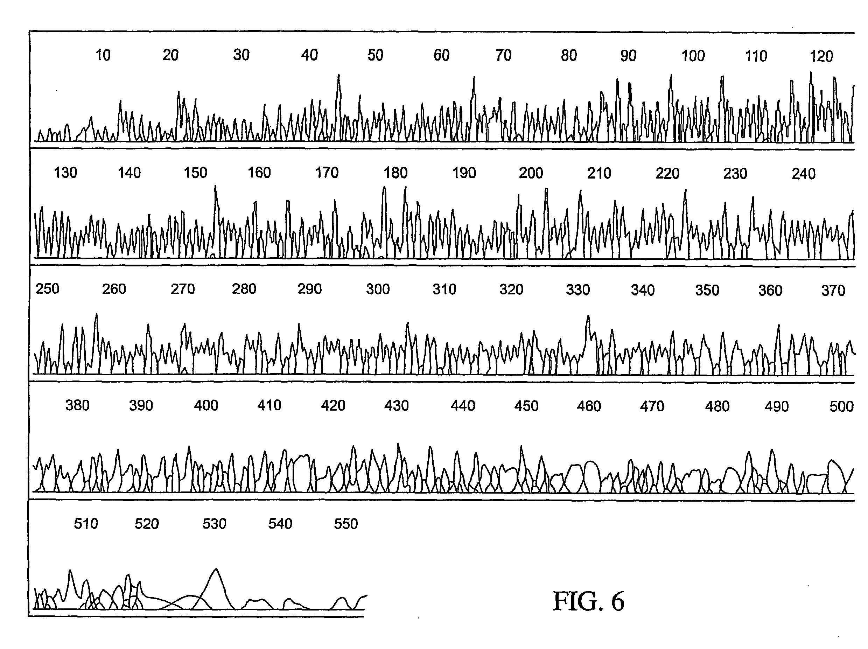

Fig. 6 illustrated the results from this purification. Fig. 6 illustrates that the synthetic polymer-polyelectrolyte-coated particles provided desirable purification. For example, poly(AA-co-DMA) polyelectrolyte-coated particles provided substantial removal of dye blob (residual dye-labeled ddNTPs) and relatively high signal strength indicating a desirable desalting of the sample.

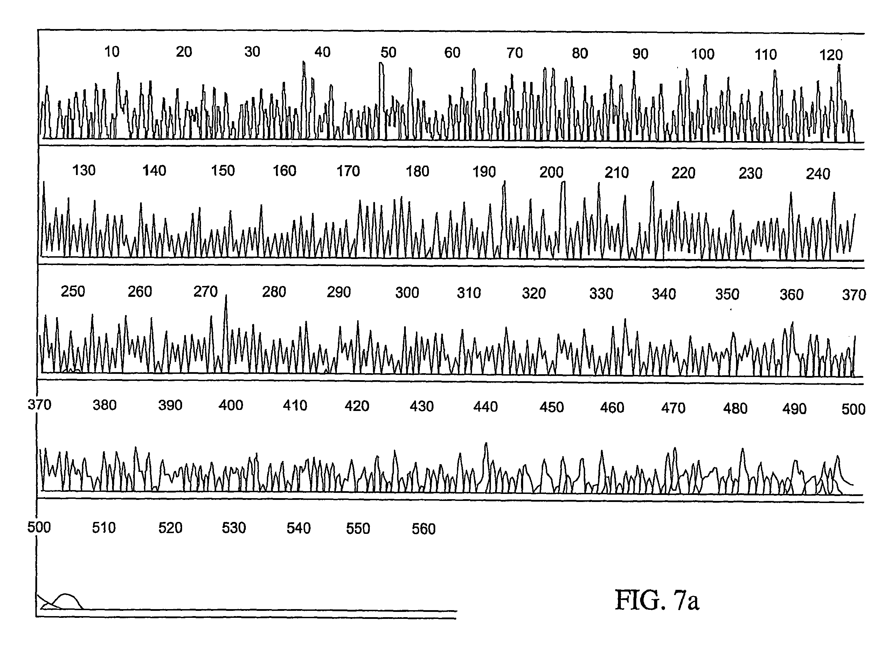

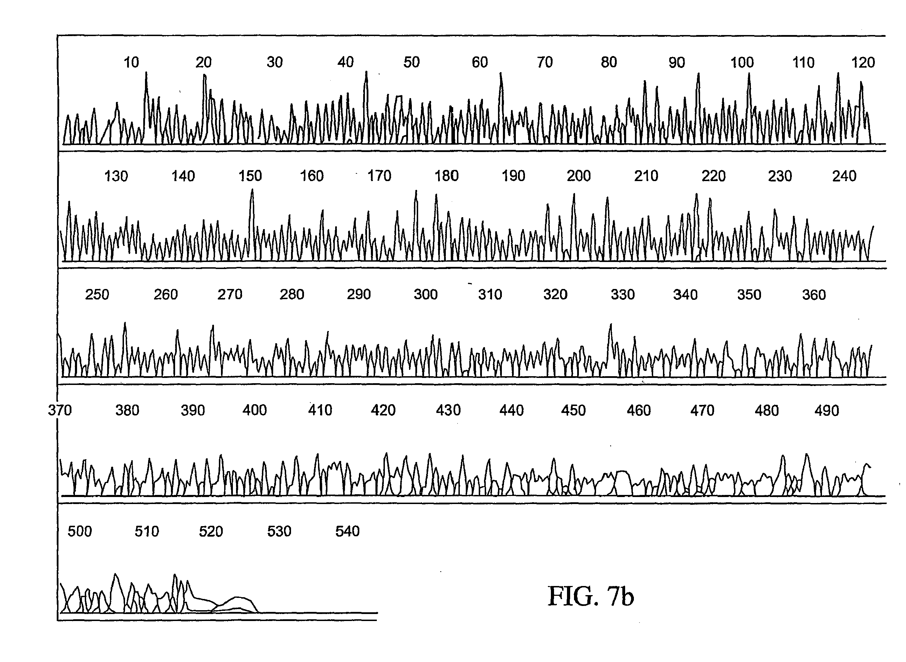

Other dRhodamine sequencing reaction products were purified by polyelectrolyte-coated particles. Fig. 7a illustrates the purification of sequencing reaction products by Bio-Rad AG 1-X8 ion-exchange resin coated with poly(AA-co-DMA) prepared as described herein Fig. 7b illustrates the purification of sequencing reaction products by Aminex A-27 ion-exchange resin coated with poly(AA-co-DMA) prepared as described herein The polyelectrolyte-coated particles provided substantial removal of dye blob (residual dye-labeled ddNTPs) and relatively high signal strength indicating a desirable desalting of the sample. Other ion-exchange resins, including Nucleosil, Isolute, Chromalite 30 SBG, Purolite-Chromalite, Macro-Prep Hi-Q, Bio-Rad AG 2-X8, Bio-Rad AG 1-X8, Aminex A-27, Powdex -PAO were tested with a variety of synthetic polymer polyelectrolytes for sequencing reaction product purification including poly(AA), poly(AA-co-AAm), poly(AA-co-DMA), poly(AA-co-PEOacrylate), poly(styrenesulfonic acid), poly(styrenesulfonic acid-co-DMA), and poly(AA-PEOacrylate-VSA).

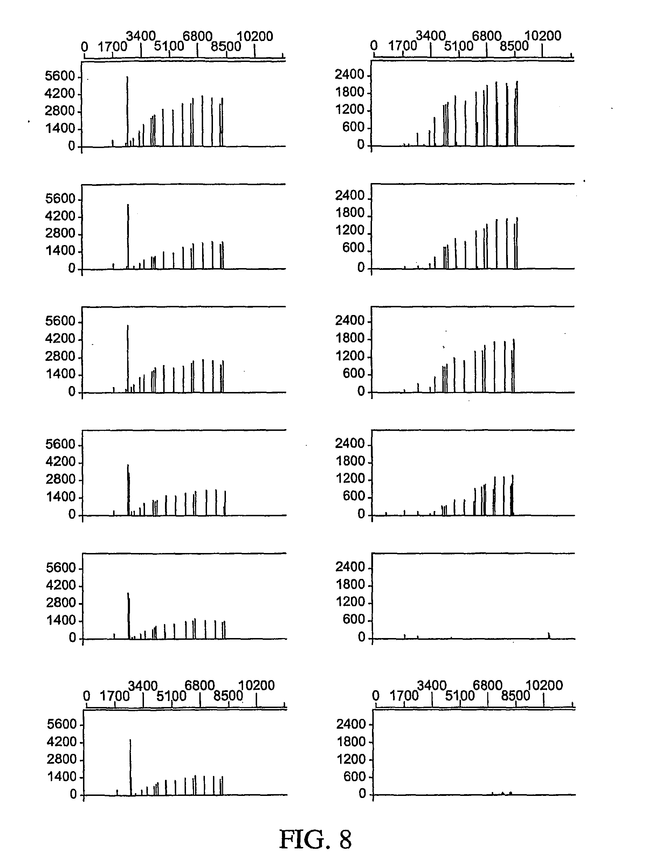

Fig. 8 illustrates the purification of sequencing reaction products purified by polyelectrolyte-coated particles coated with poly(AA-co-DMA). The sequencing reaction products were purified with different molecular weights of poly(AA-co-DMA) that increase from bottom to top electrophorograms for both the first column and second column. Sizing discrimination of polyelectrolyte-coated particles was evaluated using an assay based on the GeneScan® 500 ROX reagent (Applied Biosystems). The size standard consists of 16 dsDNA fragments ranging in size from 35bp to 500bp. The assay was performed by adding 5uL of the GeneScan® 500 ROX reagent to 5 ul of polyelectrolyte-coated particles. The mixture was agitated or vortexed for 5 minutes and the liquid is separated from the polyelectrolyte-coated particles. Separation of the polyelectrolyte-coated particles from the liquid was accomplished by centrifuging the mixture and pipetting the supernatant, or by filtration of the mixture. The resulting liquid was then analyzed using a DNA sequencer. Results of such an assay are shown in Fig. 8. The first column of Fig. 8 represents electrophorograms after separation by coated resins with pore sizes of 10 Angstroms to 15 Angstroms. The second column represents electrophorograms after separation by coated resins of pore size of 1000 Angstroms. The largest peak observed early in the electrophorogram is a primer peak and represents a fragment of 25 nucleotides. The electrophorograms in the first column of Fig. 8 shows no removal of small fragments for any of the molecular weights of coating, these electrophorograms are similar to the untreated control. Electrophorograms in the second column of Fig. 8 shows the elimination of the earlier peaks (smaller fragments) after separation with a lower molecular weight coating. These data demonstrate that the size cutoff for the polyelectrolyte-coated particles in the second column of Fig. 8 is 100bp, while the size cutoff of the polyelectrolyte-coated particles in the first column of Fig. 8 is less than 35bp. Fig. 8 illustrates the size cutoff for separation by the polyelectrolyte-coated particles for purification of sequencing reaction products.

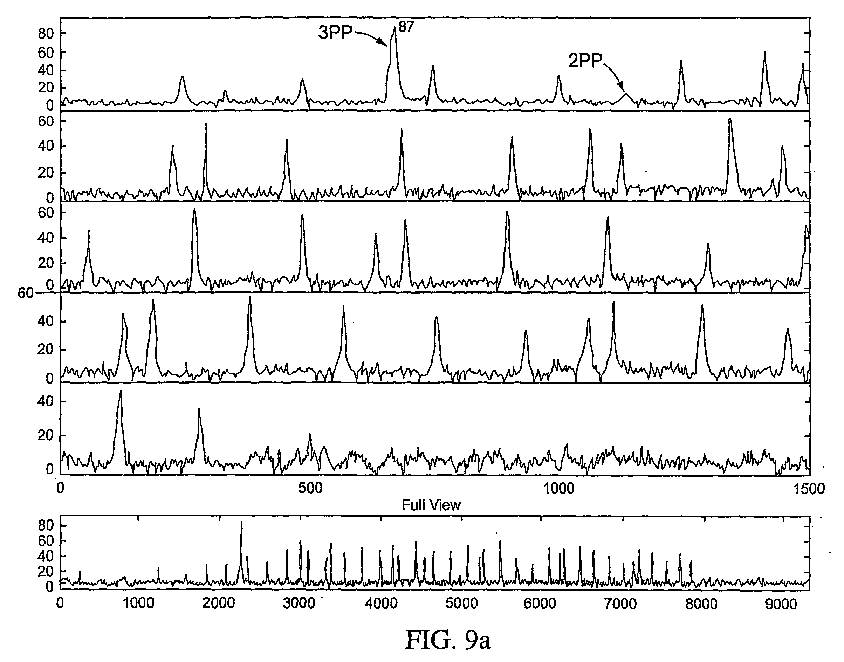

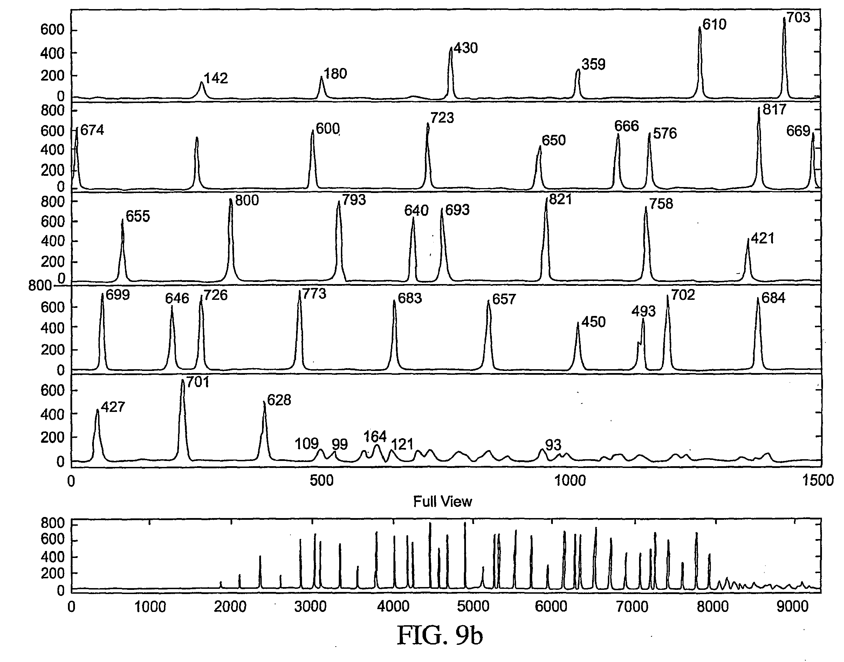

Figs. 9a and 9b illustrates the purification of sequencing reaction products purified by polyelectrolyte-coated particles including Powdex-PAO ion-exchange resin coated with poly(AA-co-DMA). Fig. 9a illustrates an electrophorogram taken before purification and Fig. 9b illustrates an electrophorogram taken after purification. The polyelectrolyte-coated particles removed LIZ® dye dTDP (2PP) and LIZ® dye dTTP (3PP) from the sequencing reaction products as labeled on Fig. 9a. The polyelectrolyte-coated particles removed other anions from the sequencing reaction products and improved electrokinetic injection of the sequencing reaction products, resulting in a factor of ten increase in signal strength.

### PCR REACTION PURIFICATION BY POLYELECTROLYTE-COATED PARTICLES WITH SYNTHETIC POLYMER:

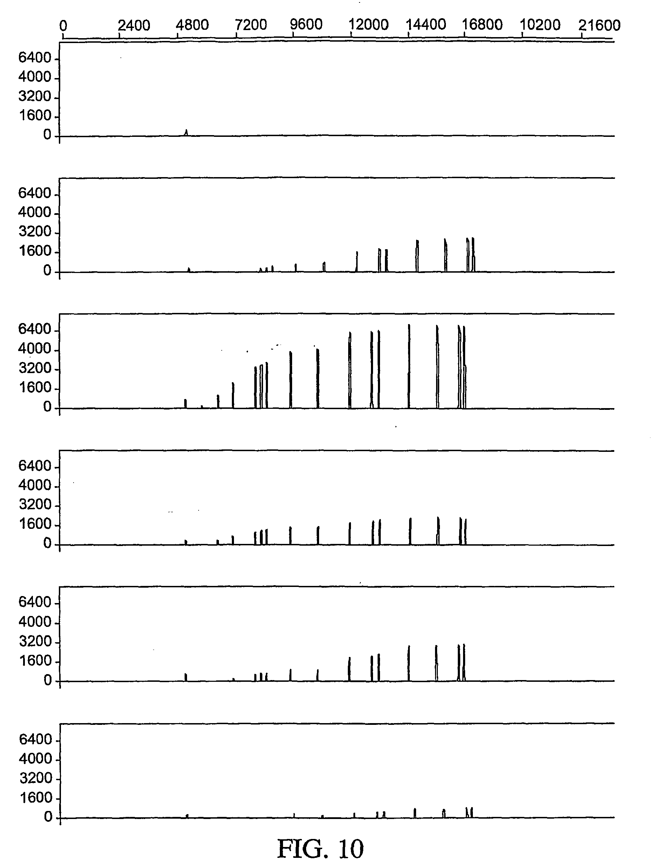

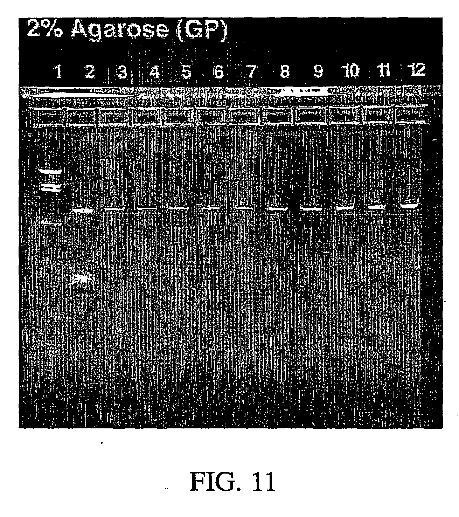

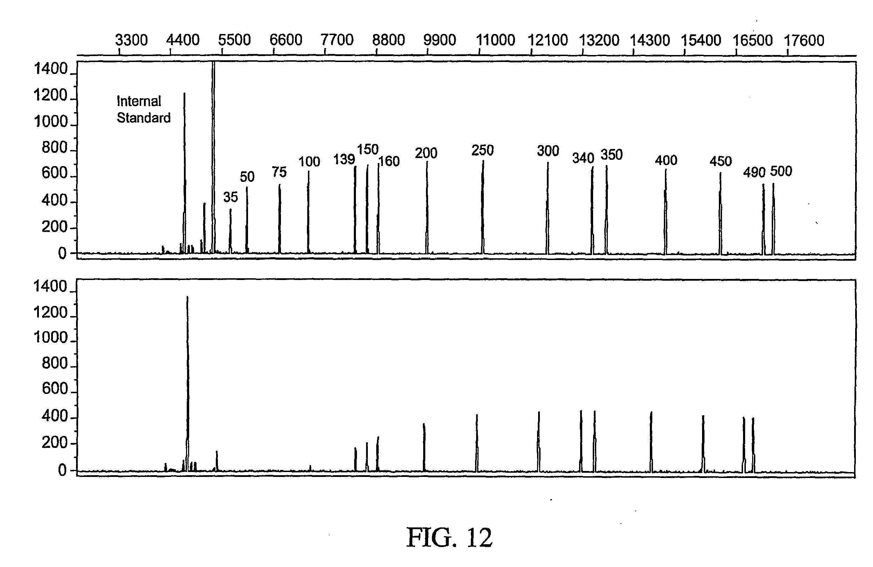

PCR reaction product purification was provided by polyelectrolyte-coated particles with synthetic polymer. Macro-Prep HQ ion-exchange resin was coated with poly(AA-co-DMA). Fig. 10 illustrates varying molar percentage of acrylic acid and molecular weigh of the poly(AA-co-DMA). The molecular weight and molar percentage increase from bottom to top from the bottom electrophorogram representing separation with resin coated with 1.1 mol% acrylic acid and 98.9 mol% DMA to the top electrophorogram representing separation with resin coated with 100 molar percent of acrylic acid or poly(AA) without N,N-dimethylacrylamide (DMA). Polyelectrolyte-coated particles containing 100 % acrylic acid removed primers, primer-dimer, and all DNA fragments. The same phenomenon was observed with poly(AA-co-DMA) containing a low acrylic acid content, 1.1 mol% acrylic acid. Fig. 11 illustrates the removal of oligonucleotide primers, primer-dimer and DNA fragments by non-desalting Macro-Prep 50 HQ (chloride form) ion-exchange resin coated with poly(AA-co-DMA) in the ranges described above. Lane 1 of Fig. 11 was loaded with the size standard, lane 2 was loaded with the one microliter of raw (no separation with polyelectrolyte coated ion-exchange particles) PCR product with 20 micromolar of primer, lanes 3-7 were loaded with one microliter of PCR product after separation with polyelectrolyte-coated ion-exchange particles, and lanes 8-12 were loaded with two microliters of PCR product after separation with polyelectrolyte-coated ion-exchange particles. Lane 2 shows the unseparated PCR products such as primers, primer-dimer, etc. as a diffuse band below the main band. Lanes 3-12 do not have such as corresponding band. Fig. 12 illustrates the size-based removal of primer-dimer and non-specifically amplified dsDNA from PCR products by non-desalting Macro-Prep 50 HQ (chloride form) ion-exchange resin coated with poly(AA-co-DMA) in the ranges described above. The upper electrophorogram shows PCR products with no separation with polyelectrolyte coated ion-exchange particles. The lower electrophorogram shows PCR products separated with polyelectrolyte coated ion-exchange particles. The difference illustrates that dsDNA fragments smaller than 100bp were separated from larger fragments that remain in solution after separation with polyelectrolyte coated ion-exchange particles.

### DNA SEQUENCING REACTION PURIFICATION BY POLYELECTROLYTECOATED PARTICLES WITH SYNTHETIC POLYMER AND SURFACTANTS:

Surfactants can be added to DNA sequencing reactions prior to purification using size-based purification methods. For example, prior to purification of DNA sequencing reactions using size-exclusion spin columns, sodium dodecyl sulfate (SDS) can be added to assist in purification. Further, the DNA sequencing reaction including SDS can be heated prior to purification using size-exclusion spin columns. In various embodiments, anionic surfactants can be replaced with non-ionic surfactants in DNA sequencing reaction purified by polyelectrolyte-coated ion-exchange particles. Such replacement can prevent interaction of an ionic surfactant and the ion-exchange particle to avoid adverse effects on the ion-exchange capacity of the polyelectrolyte-coated ion-exchange particles. The term "non-ionic" surfactant as used herein refers to non-ionic surfactants, zwitter-ionic surfactants, or other surfactants that do not substantially contribute to the conductivity of the solution. Examples of non-ionic surfactants include Brij 35 and Brij 58 (CalBiochem, San Diego, CA), Triton X-100 (Sigma, St. Louis, MO), Octyl-β-D-glucoside, octylglucopyranoside, and (3-[(3-Cholamidopropyl) dimethylammonio]-1-propanesulfonate) ("CHAPS") (Pierce Biotechnology, Rockford, IL). In various embodiments, the non-ionic surfactants can be activatable without heating in order to facilitate the removal of unicorporated dye-terminators from the DNA sequencing reaction.

For DNA sequencing reaction purification, a sample was prepared using fluorescently-labeled dideoxy nucleotide terminators so that the sequencing reaction product could be analyzed on a fluorescent capillary sequencer. A solution was prepared containing 20 uL using a 8 uL of Big Dye Terminator® v.3.1 Ready Reaction Mix (Applied Biosystems, Foster City, California). The sequencing primer was M13 universal reverse primer (3.2 pmol/uL). The template was a plasmid, pGEM (200 ng/uL). This master solution was aliquoted into wells in a GeneAmp® 9700 thermal cycler plate. The mixture was subjected to 25 cycles of heating, wherein each cycle included heating at 96°C for 10 seconds, heating at 50°C for 5 seconds, and heating at 60°C for 240 seconds.

After pooling, 10 uL of the pooled, unpurified reaction mixture was redistributed to each of 16 wells of a new 96 well MicroAmp® tray for purification. Each well contained the 10 uL of reaction mixture, 20 uL of de-ionized water, 10 uL of surfactant and 10 uL of polyelectrolyte-coated ion-exchange particle slurry including poly(styrenesulfonic acid)-coated AG1X-8 in a mixed bed with AG50WX-8 prepared as described above. The final volume of the mixture was 45 uL because of the polyelectrolyte-coated ion-exchange slurry was 50% water. In these examples, two different surfactant solutions were used: (1) 10 uL of Brij 35 aqueous solution at three percent by volume, final concentration of Brij 35 in entire solution was 0.67%; (2) and 10 uL of Triton X-100 aqueous solution at 10 percent by volume, final concentration of Triton X-100 in entire solution was 2.2%. Controls for each of the surfactants replaced the surfactant with 10 uL of water. In various embodiments, surfactants can be effective at concentrations ranging from 0.01% to 10%. In various embodiments, surfactants can be effective at concentrations ranging from 0.1 % to 5%.

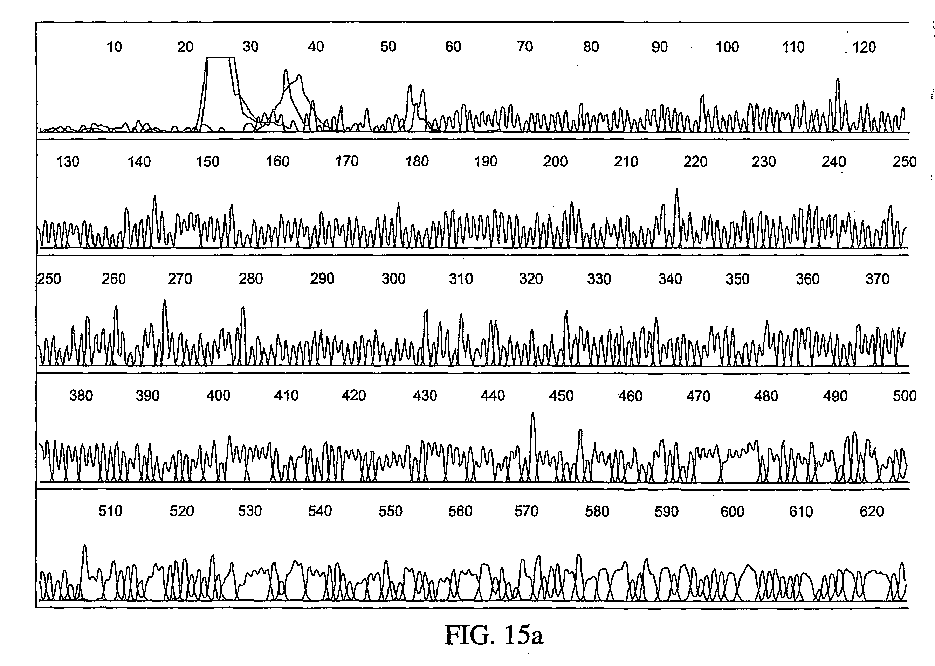

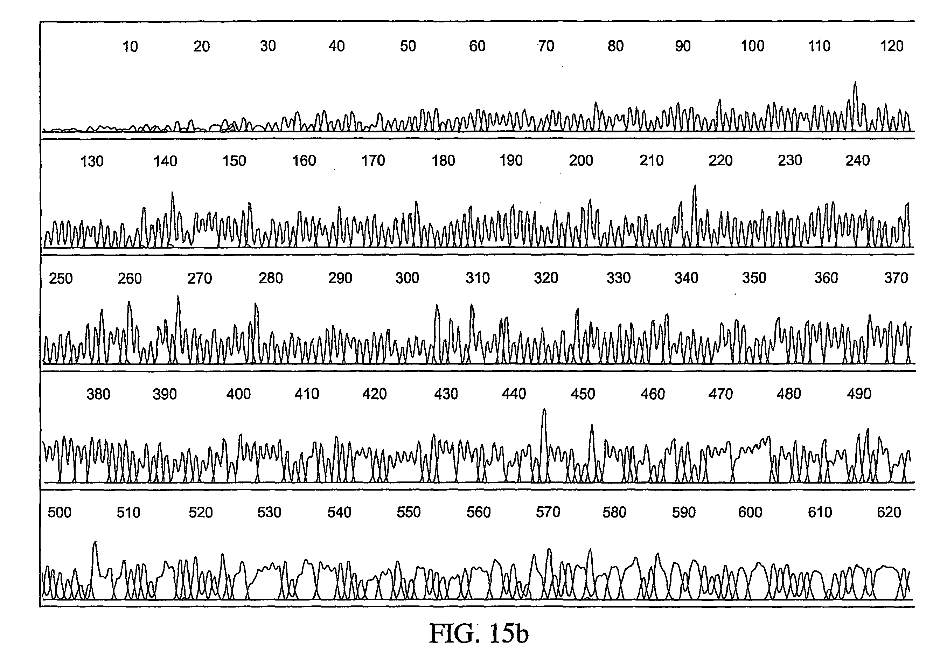

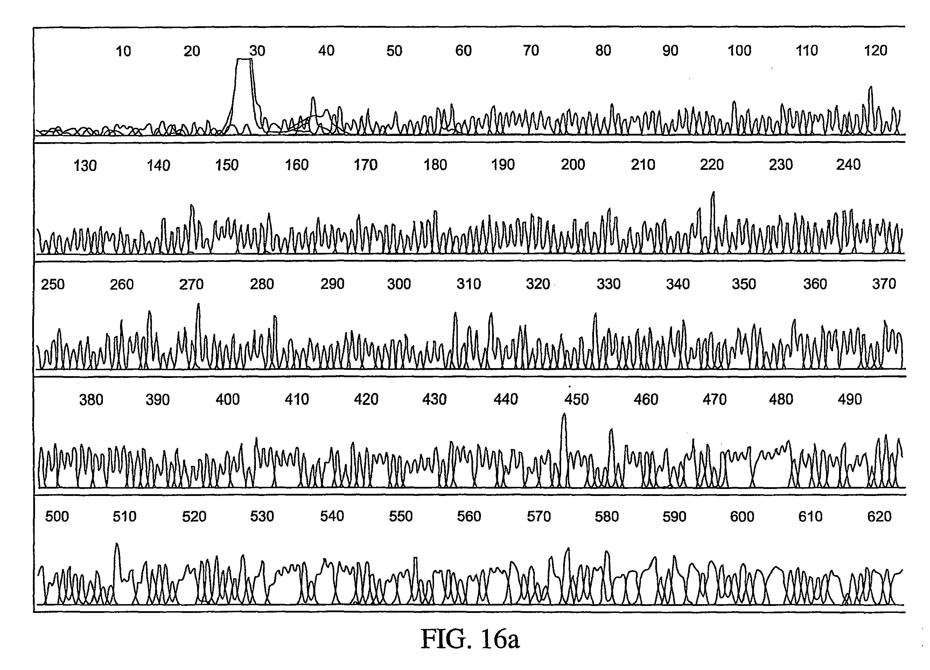

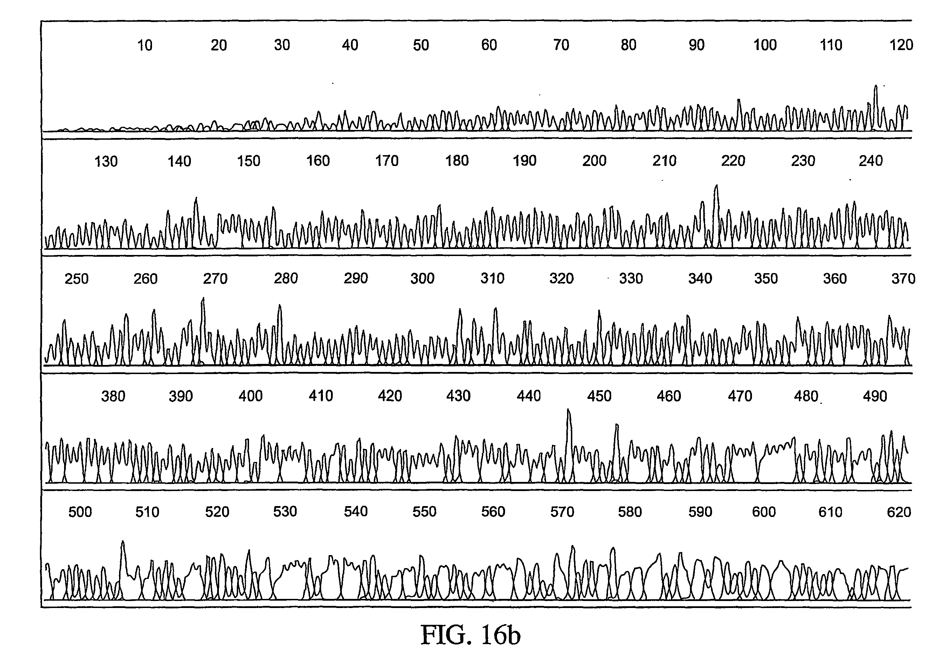

The samples in the MicroAmp® tray were then covered with an adhesive film and mixed with a vortexer for 30 minutes. Following mixing, the MicroAmp® tray containing the samples was centrifuged at 3000 rpm for five minutes. The MicroAmp® tray was then transferred to an ABI Prism® 3100 Genetic Analyzer for DNA sequencing. The DNA sequencing is carried out with a 50 cm capillary array and POP-6® capillary electrophoresis polymer. The injection conditions were 1000V at 22 seconds directly from the supernatant in the tube containing the sample and the beads as described below. Figs. 15A and 15B illustrate DNA sequencing purification with and without Brij 35 surfactant, respectively. In Fig. 15A, purified without Brij 35 surfactant, dye artifacts appeared, for example, in the region from 15-60 nucleotides of the sequence. In Fig. 15B, purified with Brij 35 surfactant, dye artifacts in that same region did not appear. Figs. 16A and 16B illustrate DNA sequencing purification with and without Triton X-100 surfactant, respectively. In Fig. 16A, purified without Triton X-100 surfactant, dye artifacts appeared, for example, in the region from 15-60 nucleotides of the sequence. In Fig. 16B, purified with Triton X-100 surfactant, dye artifacts in that same region did not appear. In both instances, the surfactant was able to facilitate removal of the dye artifacts without heating and without interfering with direct electrokinetic injection or polyelectrolyte-coated ion-exchange particle purification.

### COATING PARTICLES WITH MULTIPLE SYNTHETIC POLYMER LAYERS:

Poly(AA-co-DMA) polyelectrolyte was prepared as described above. Poly(allylamine hydrochloride), or "PAH," M_{w} 0.07 MDa and poly(acrylic acid), or "PAA," M_{w} 4.00 MDa (Aldrich Chemicals) were readily available. The following polyelectrolyte solutions (0.5 wt%) were prepared by adding the appropriate amount of polymers to the deionized waster and then tumbled overnight at ambient temperature: poly(AA-co-DMA) 0.2286g /39.915g H₂O; PAH 0.2268g /40.033g H₂O; PAA 0.1192 g / 19.99g H₂O.

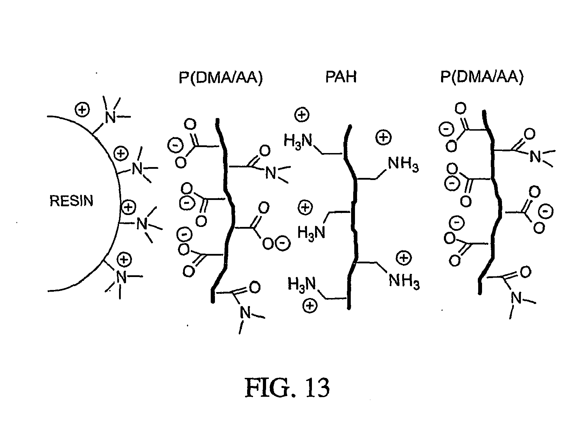

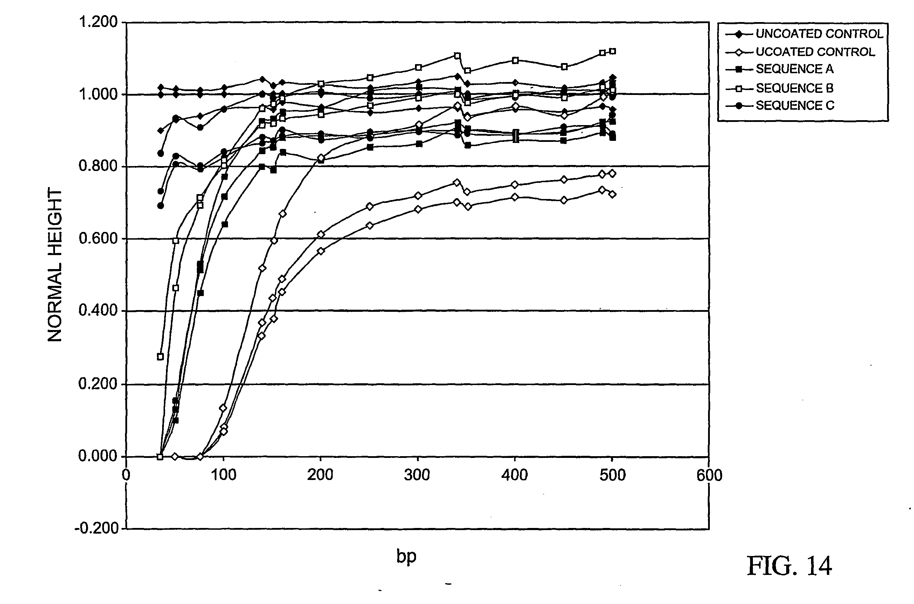

Prior to coating, the anion-exchange resin (Macro-Prep High Q 50, Bio-Rad) was washed with de-ionized water three times. A slurry solution of resin (3 mL, 0.35g of wet resin) and 20 mL of de-ionized water were added to a 50 mL tube. To coat the resin, the polyelectrolyte solution (0.750 mL) was added. The tube was vortexed for one minute, spun at 1760 rpm for three minutes, and the supernatant was decanted. The wet resin was dried under high vacuum oven at 70 degrees centigrade for two hours. This coating process was repeated two more times with subsequent polyelectrolyte solutions to apply a total of three polyelectrolyte coating layers. For the last drying step, the coating was dried overnight. The dried pellet was mixed by vortexing with 40 mL of de-ionized water, spun at 1670 rpm for three minutes, and the supernatant was removed. The final washed resin was redispersed in 10 mL of de-ionized water and stored in a refrigerator prior to use. Fig. 13 illustrates the multiple polyelectrolyte coatings applied to the resin in one of the examples. Fig. 14 illustrates the performance of an uncoated resin, a coated resin, and several trials of three sequences of multiple polyelectrolyte coatings on the ion-exchange resin by showing removal of smaller numbers of base pairs (bp). The three sequences of polyelectrolytes are described in Table 1 below, with the first layer being closest to the surface of the resin and subsequent layers after that.

**Table 1.**

| | Sequence A | Sequence B | Sequence C |
|---|---|---|---|
| First Layer | Poly(AA-co-DMA) | Poly(AA-co-DMA) | PAA |
| Second Layer | Poly(AA-co-DMA) | PAH | PAH |
| Third Layer | Poly(AA-co-DMA) | Poly(AA-co-DMA) | PAA |

### MAGNETIZATION OF POLYELECTROLYTE COATED RESIN:

PAA M_{w} 4.00 MDa (Aldrich Chemicals) and Fe3O₄ (EMG 1111) (Ferrotec Corp.) were readily available. A solution of PAA (0.750 mL) was added to a slurry solution of resin (0.2g of wet resin) and 10 mL de-ionized water. The mixture was vortexed for one minute, incubated at ambient temperature for five minutes, spun at 1760 rpm for three minutes, and the supernatant was decanted. The resin was washed once with de-ionized water (10 mL). The suspension was spun at 1760 rpm for three minutes and water was decanted. An aqueous solution of Fe₃O₄ (0.20 mL) with de-ionized water was added to the wet resin. The suspension was mixed by vortex for one minute and incubated at ambient temperature for five minutes. The suspension was spun at 1670 rpm for three minutes and the aqueous solution was removed. The resin was washed three times to remove excess unbound Fe₃O₄. The coated resin was redispersed in de-ionized water (10 mL). Magnetization of the resin was observed.

### SEPARATION OF POLYELECTROLYTE-COATED ION-EXCHANGE PARTICLES

Within each category of reactions and/or examples provided above, there are numerous specific applications. For instance, PCR reaction polyelectrolyte-coated ion-exchange particles can provide purification for sequence detection applications (both end-point and real-time), PCR cloning applications, etc., and DNA sequencing reaction polyelectrolyte-coated ion-exchange particles can provide purification for any type of nucleic acid sequencing, for example, sequencing, fragment analysis, single nucleotide polymorphism identification, etc.

It can be desirable to separate the polyelectrolyte-coated ion-exchange particles from the purified supernatant liquid before sequencing the nucleic acids. Sequencing can be achieved by, for example, capillary electrophoresis. Isolating the purified supernatant liquid from the polyelectrolyte-coated ion-exchange particles can add steps such as pipetting, sample transfers, centrifugation, magnetic removal and/or thermal cycling between purification and sequencing. It is desirable to eliminate such steps to increase efficiency and speed, and reduce cost and hands-on time for the user between purification and sequencing.

According to various embodiments, isolation of the polyelectrolyte-coated ion-exchange particles from the purified supernatant liquid can be achieved by filtration to retain the particles or centrifugation to collect the particles followed by aspiration of the supernatant liquid. It is desirable to eliminate the step of removing the polyelectrolyte-coated ion-exchange particles from the purified supernatant liquid by loading the sample for capillary electrophoresis directly from the supernatant liquid. However, the polyelectrolyte-coated ion-exchange particles can interfere with loading by inhibiting electrokinetic injection and causing random disruption in sequencing data or by clogging the capillary when polyelectrolyte-coated ion-exchange particles are crushed by the capillary against the bottom of the sample tube.

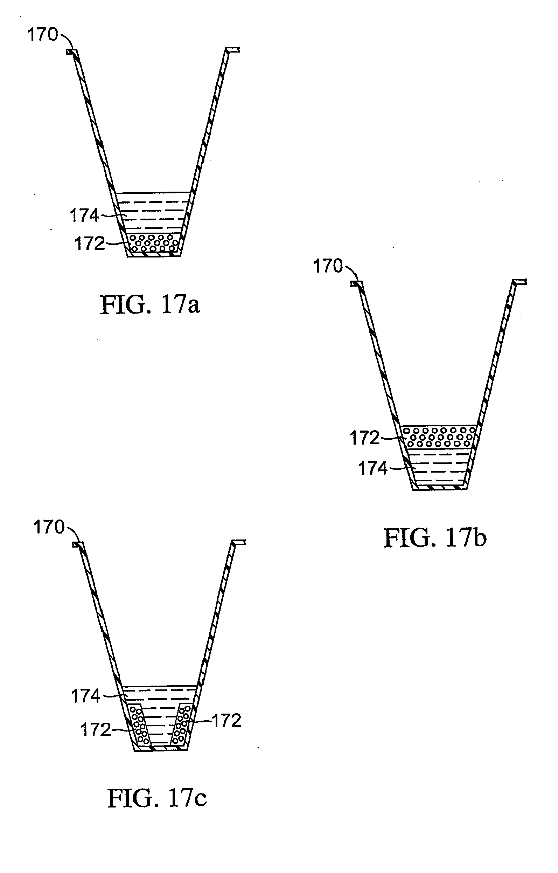

According to various embodiments, the present teachings provide a method for loading the capillary from the bottom of the sample well, tube, or chamber. The polyelectrolyte-coated ion-exchange particles can be moved to the sides or the top of the well. Figs. 17A-17C illustrate a well 170 where the polyelectrolyte-coated ion-exchange particles 172 have been isolated from the supernatant liquid 174. The teachings provide positioning the polyelectrolytecoated ion-exchange particles 172 at the top of the well 170 as illustrated in Fig. 17B or positioning the polyelectrolyte-coated ion-exchange particles 172 at the sides of well 170 as illustrated in Fig. 17C.

According to various embodiments, the polyelectrolyte-coated ion-exchange particles can be positioned at the top of well by floating with a higher density solvent. The supernatant liquid's density can be increased such that it has greater density than the polyelectrolyte-coated ion-exchange particles. Examples of solvents that can increase the density of the supernatant include ethylene glycol (density = 1.113), propylene carbonate (density = 1.189), polyethylene glycol (a waxy solid with density = 1.127), glycerol (density = 1.261), and combinations thereof at various concentrations. In various embodiments, ion-exchange resins can have densities down to the 1.03 level. Upon increasing the density of the supernatant liquid, the polyelectrolyte-coated ion-exchange particles float to the top of the well. The capillary and electrode can then be immersed in the well below the layer of polyelectrolyte-coated ion-exchange particles. The loading of the capillary can be free of the polyelectrolyte-coated ion-exchange particles. The addition of the solvent eliminates centrifugation step in sample preparation and can be automated by an array pipettor.

According to various embodiments, the polyelectrolyte-coated ion-exchange particles can be positioned at the sides of the well by magnetic attraction. As described above, the polyelectrolyte-coated ion-exchange particles can be coated with Fe₃O₄ to provide magnetization. The polyelectrolyte-coated ion-exchange particles can be attracted to the sides of the well by a magnetic field on a side of the well or around the well provided by a magnet or an electrically induced magnetic field. The capillary and electrode can then be immersed in the center of the well clear of the concentric layer or beside the layer of polyelectrolyte-coated ion-exchange particles. The loading of the capillary can be free of the polyelectrolyte-coated ion-exchange particles. The magnetization of the polyelectrolyte-coated ion-exchange particles eliminates centrifugation step in sample preparation.

According to various embodiments, the present teachings provide a method for loading the capillary from the top of the sample well, tube, or chamber. The polyelectrolyte-coated ion-exchange particles can be moved to the bottom of the well. Fig. 17A illustrates a well 170 where the polyelectrolyte-coated ion-exchange particles 172 have been isolated from the supernatant liquid 174. The particles can be moved to the bottom by centrifugation or magnetization as discussed above. A sequencer instrument can be calibrated to load the sample from a higher point in the well free of the polyelectrolyte-coated ion-exchange particles. Modification of the capillary position for supernatant loading can be provided by increasing the distance between the capillary tip to the bottom of the tube. For standard microtiter trays, an example of such a distance is 4.5 mm. This corresponds to a well volume for a 96-well tray of 30 uL. The volume of the supernatant can be increased to insure that the capillary is immersed in supernatant for good electrokinetic injection. This can be done by increasing the volume of the supernatant by addition of de-ionized water. The mixture of the de-ionized water and the supernatant can be provided by vortexing the mixture for 30 minutes.

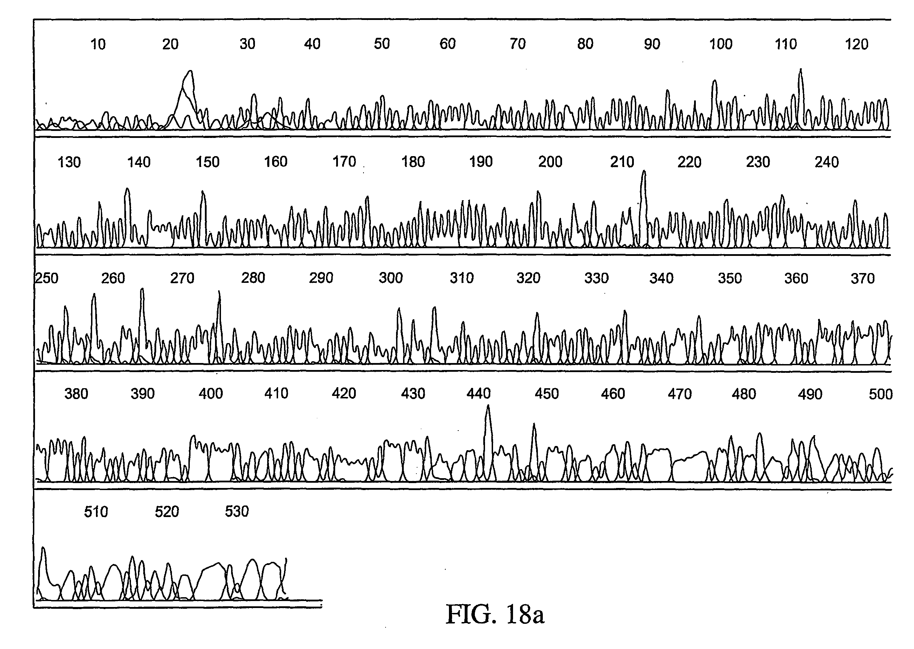

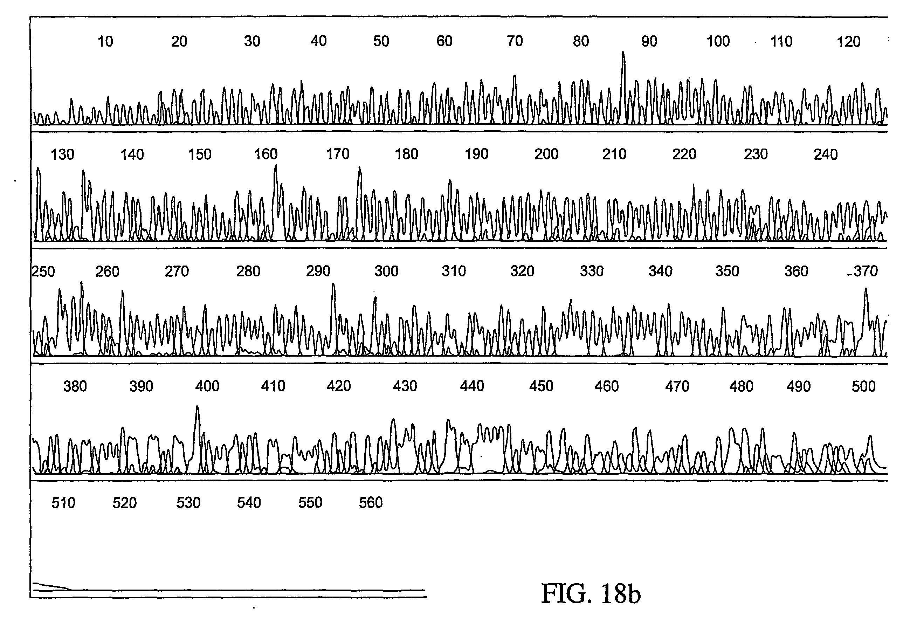

According to various embodiments, the sequencer instrument can be calibrated to load the sample from a higher point in the well by mechanically changing the tray or frame into which the tray mates so that the tray sits lower in the instrument. According to various embodiments, the sequencer instrument can be calibrated to load the sample from a higher point in the well by modifying the firmware initialization file to change the z-axis point-of-reference. According to various embodiments, the sequencer instrument can be calibrated to load the sample from a higher point in the well by programming software to retract the capillary from the bottom loading position to a point in the supernatant liquid layer above the polyelectrolyte-coated ion-exchange particles. Figs. 18A-18B illustrate nucleic acid sequencing from supernatant using Big Dye Terminator® chemistry (Fig. 18A) and dRhodamine terminator chemistry (Fig. 18B) using a sequencer instrument calibrated to load the sample from a higher point in the well by modifying the firmware initialization file to change the z-axis point-of-reference.

For the purposes of this specification and appended claims, unless otherwise indicated, all numbers expressing quantities of ingredients, percentages or proportions of materials, reaction conditions, and other numerical values used in the specification and claims, are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Moreover, all ranges disclosed herein are to be understood to encompass any and all subranges subsumed therein. For example, a range of "1 to 10" includes any and all subranges between (and including) the minimum value of 1 and the maximum value of 10, that is, any and all subranges having a minimum value of equal to or greater than 1 and a maximum value of equal to or less than 10, e.g., 5.5 to 10.

It is noted that, as used in this specification and the appended claims, the singular forms "a," "an," and "the," include plural referents unless expressly and unequivocally limited to one referent. Thus, for example, reference to "a monomer" includes two or more monomers. Furthermore, the use of the term "including", as well as other forms, such as "includes" and "included", is not limiting.

It will be apparent to those skilled in the art that various modifications and variations can be made to various embodiments described herein without departing from the spirit or scope of the present teachings. Thus, it is intended that the various embodiments described herein cover other modifications and variations within the scope of the appended claims and their equivalents.

## Claims

1. A particle comprising:
a core comprising ion-exchange material; and
a coating comprising polyelectrolyte material,
wherein the core and coating are adapted to separate PCR reaction products or DNA sequencing reaction products, and the coating covers the entire exterior surface of the particle.

2. The particle of claim 1, wherein the core couples to at least one PCR reaction product chosen from primers, primer-dimer, ssDNA fragments, unincorporated nucleotides, and salts.

3. The particle of claim 2, wherein the particle is adapted to substantially exclude dsDNA fragments having greater than 100 basepairs.

4. The particle of claim 1, wherein the core couples to at least one DNA sequencing reaction product chosen from primers, dye-labeled primers, nucleotides, dye-labeled nucleotides, dideoxynucleotides, dye-labeled dideoxynucleotides, and salts.

5. The particle of claim 4, wherein the particle is adapted to substantially exclude dye-labeled ssDNA fragments having greater than 45 nucleotides.

6. The particle of claims 1-5, wherein the coating comprises a biopolymer, wherein the biopolymer is non-sample DNA.

7. The particle of claims 1-5, wherein the coating comprises a synthetic polymer.

8. The particle of claim 7, wherein the synthetic polymer comprises a copolymer, wherein the copolymer comprises at least one monomer chosen from (meth)acrylamide, N-methyl (methyl)acrylamide, N,N-dimethyl (methyl)acrylamide, N-ethyl (meth)acrylamide, N-n-propyl (meth)acrylamide, N-iso-propyl (meth)acrylamide, N-ethyl-N-methyl (meth)acrylamide, N,N-diethyl (meth)acrylamide, N-hydroxymethyl (meth)acrylamide, N-(3-hydroxypropyl) (methy)acrylamide, N-vinylformamide, N-vinylacetamide, N-methyl-N-vinylacetamide, vinyl acetate (precursor of vinyl alcohol), 2-hydroxyethyl (meth)acrylate, 3-hydroxypropyl (meth)acrylate, N-vinypyrrolidone, poly(ethylene oxide) (methy)acrylate, N-(meth)acryloxysuccinimide, N-(meth)acryloylmorpholine, N-2,2,2-trifluoroethyl (meth)acrylamide, N-acetyl (meth)acrylamide, N-amido(meth)acrylamide, N-acetamido (meth)acrylamide, N-tris(hydroxymethyl)methyl (meth)acrylamide, styrenesulfonic acid, homopolymers of styrenesulfonic acid, co-polymers of styrenesulfonic acid, N-(methyl)acryloyltris(hydroxymethyl)methylamide, (methyl) acryloylurea, vinyloxazolidone, vinylmethyloxazolidone, acrylic acid, methacrylic acid, vinyl sulfonic acid, styrene sulfonic acid, 4-acetoxystyrene (precursor of 4-hydroxystyrene), and vinylphosphonic acid, and vinyl methyl ether.

9. The particle of claim 8, wherein the synthetic polymer is poly(acrylic acid-co-N,N-dimethylacrylamide) or poly(N,N-dimethyl acrylamide-co-styrene sulfonic acid).

10. The particle of claim 1, wherein the ion-exchange material is porous.

11. The particle of claim 10, wherein the ion-exchange material is surface-activated.

12. The particle of claim 10, wherein the ion-exchange material has a pore size of 100 Angstroms to 2000 Angstroms or 5 Angstroms to 1000 Angstroms.

13. The particle of claim 12, wherein the polyelectrolyte material has a Mw of 1000 Daltons to 6.0 megaDaltons.

14. The particle of claim 12, wherein the ion-exchange material has the pore size of 1000 Angstroms and the Mw of 1.7 megaDaltons to 2.4 megaDaltons or the pore size of 10 Angstroms to 50 Angstroms and the Mw of 2.4 megaDaltons to 4.9 megaDaltons.

15. The particle of claim 8, wherein the synthetic polymer is poly(N-(3-aminopropyl)methacrylamide-co-N,N-dimethylacrylamide).

16. The particle of any of the preceding claims, wherein the polyelectrolyte material comprises multiple layers.

17. The particle of claim 16, wherein the multiple layers comprise polyanions and polycations is alternating layers.

18. The particle of claim 17, wherein the core is macroporous and each of the multiple layers of polyelectrolyte has a molecular weight capable of penetrating the macroporous core.

19. A mixture comprising particles of claim 1, and a cationic ion-exchange material or an anionic ion-exchange material.

20. The mixture of claim 19, wherein the cationic ion-exchange material or the anionic ion-exchange material form a mixed bed with the particle.

21. The mixture of claim 19, further comprising a non-ionic surfactant.

22. The particle of claim 1 wherein the polyelectrolyte material is a polyanion comprising carboxylic functional groups, boric functional groups, sulfonic functional groups, sulfinic functional groups, phosphoric functional groups, phosphorus functional groups, phenolic functional groups, or a combination thereof.

23. The particle of claim 7, wherein the polymer is a homopolymer selected from homopolymers of styrene sulfonic acid, acrylic acid, methacrylic acid, vinyl sulfonic acid, vinyl phosphonic acid, allyl amine hydrochloride, (3-acrylamidopropyl) trimethylammonium chloride, N-(3-aminopropylmethacrylamide hydrochloride, fumaric acid, aspartic acid, galactronic acid, glutamic acid, adenylic acid, inosinic acid, uridylic acid and polysaccharides.

24. The particle of claim 12, wherein the polyelectrolyte material has a Mw of 1.0 megaDaltons to 3.0 megaDaltons.

25. A device comprising a receptacle for purification wherein the mixture of claim 19 is disposed in the receptacle or a microfluidic device comprising a plurality of columns wherein the mixture of claim 19 is disposed in each column.
